(19) Europäisches Patentamt / European Patent Office / Office européen des brevets

(11) **EP 3 982 978 B1**

(12) **EUROPEAN PATENT SPECIFICATION**

(45) Date of publication and mention
of the grant of the patent:
**22.04.2026 Bulletin 2026/17**

(21) Application number: **20733941.7**

(22) Date of filing: **17.06.2020**

(51) International Patent Classification (IPC):
*A61K 31/734* (2006.01)    *A61P 7/02* (2006.01)

(52) Cooperative Patent Classification (CPC):
**A61P 7/02; A61K 31/734; A61K 45/06**

(86) International application number:
**PCT/EP2020/066799**

(87) International publication number:
**WO 2020/254427 (24.12.2020 Gazette 2020/52)**

(54) **ALGINATE OLIGOMERS FOR USE IN THE ANTICOAGULATION THERAPY OF SUBJECTS AT RISK OF BLOOD CLOTS WHICH HAVE AN ABNORMALLY DENSE MICROSTRUCTURE**

ALGINATOLIGOMEREN ZUR VERWENDUNG IN DER ANTIKOAGULATIONSTHERAPIE VON PERSONEN MIT EINEM RISIKO FÜR BLUTGERINNSEL MIT ABNORMAL DICHTER MIKROSTRUKTUR

OLIGOMÈRES D'ALGINATE POUR L'UTILISATION DANS LE TRAITEMENT ANTICOAGULANT DE SUJETS À RISQUE DE CAILLOTS SANGUINS PRÉSENTANT UNE MICROSTRUCTURE ANORMALEMENT DENSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.06.2019 GB 201908639**

(43) Date of publication of application:
**20.04.2022 Bulletin 2022/16**

(73) Proprietor: **ALGIPHARMA AS**
**1337 Sandvika (NO)**

(72) Inventors:
• **WILLIAMS, Rhodri**
**Swansea West Glamorgan SA3 2AD (GB)**
• **EVANS, Phillp Adrian**
**Carmarthenshire SA32 8NW (GB)**
• **DESSEN, Arne**
**3440 Røyken (NO)**
• **RYE, Philip**
**1359 Eiksmarka (NO)**

(74) Representative: **Dehns**
**10 Old Bailey**
**London EC4M 7NG (GB)**

(56) References cited:
**WO-A1-2015/092437**

## Description

The present invention relates to alginate oligomers for use as defined in the claims as blood anticoagulants specifically in a group of subjects which are predisposed to the formation of blood clots having an abnormal, or altered, microstructure, and more particularly a density which is greater than normal. Blood clots with microstructure of increased density are stronger, less flexible, less permeable and more resistant to degradation both by endogenous (i.e. natural or internal) processes and by exogenous (i.e. external) pharmaceutical interventions and so such clots have the potential to be more detrimental to the subject in pathological thrombotic scenarios. The present invention is based on the finding that blood clots formed in the presence of alginate oligomers have a microstructure of reduced density. Accordingly the invention provides the means to normalise clot microstructure and thereby reduce the incidence of, or to inhibit (i.e. prevent or reduce) the occurrence of, blood clots which have an abnormal, e.g. denser, microstructure in subjects predisposed thereto and thereby enhance the anticoagulation therapy of such subjects.

Blood coagulation (or blood clotting, which terms are used interchangeably herein) is a physiological mechanism evolved by vertebrates to counteract and remedy injury to the circulatory system. Put simply, the blood coagulation process results in the production of an aggregated mass of cells and proteinaceous matrix that physically plugs the breech in the integrity of the circulatory system caused by the injury.

More specifically the *in vivo* coagulation process is a complex bifurcated cascade of proteolytic reactions that results in the conversion of fluid blood to a solid gel ultimately through the conversion soluble fibrinogen to insoluble fibrin. The conversion of fibrinogen to fibrin results in a matrix of proteinaceous filaments ("microstructure") which trap blood cells and other blood components. Regardless of the exact makeup of the resulting entity, it is referred to as a blood clot or clinically as a thrombus.

The *in vivo* coagulation process is bifurcated in that there are two recognised different initiation points which converge at the point of Factor X, the protein which converts prothrombin to thrombin, which in turn catalyses the conversion of fibrinogen to fibrin. One of the bifurcated coagulation pathways is referred to as the "intrinsic pathway" or the "contact activation pathway" on account of initial observations that the pathway was activated upon contact of blood with artificial surfaces. By definition, the essential mediators of this process are all present in the blood, hence "intrinsic". The other bifurcated coagulation pathway is referred to as the "extrinsic pathway" or "tissue factor pathway" on account of the requirement for initiation by an extraneous cell surface mediator "tissue factor". Tissue factor exposure occurs immediately upon tissue injury and hence the extrinsic pathway is viewed as the dominant pathway *in vivo.*

Although blood coagulation is an essential defence mechanism of vertebrate organisms against injury, unwanted or inappropriate coagulation in a clinical context gives rise to thrombotic diseases and complications in which a blood clot causes an obstruction in a blood vessel or the heart. Such events often lead to hypoxia and even infarction in the downstream tissues. Tissue or organ damage may ensue and depending on the location of the ischemia, the thrombosis or resulting tissue damage may manifest as conditions such as hypertension, low blood oxygenation, stroke, cardiac infarction, pulmonary embolism and angina.

The formation of a clot and the characteristics of the resulting clot, as well as its association with thromboembolic disease, can depend on the microstructure of the clot, in terms of the fibrin fibre network, e.g. fibre thickness, fibre length, number of branch points in the fibrin network, network density etc. Interest has developed in recent years in characterising clot microstructure, and the modulation of clot architecture, as a measure of clot formation potential or capacity, including the quality of the clot which is formed, in the treatment of thrombosis or other thromboembolic diseases.

In particular, with the emergence of new haemorheological technologies, it is has become clear that the gel point (GP) of clot formation, that is the rheological transition between an elasticoviscous fluid and a viscoelastic solid, may be accurately determined, and that certain structural parameters of an incipient clot at its gel point can be measured or calculated with sufficient accuracy to provide a quantitative indication of relative clot density and strength during incipiency and through to maturity. These parameters include (i) the fractal dimension ($d_f$), which can be viewed as an indicator of the arrangement and mechanical properties of the clot microstructure, (ii) $T_{GP}$, which is the time taken to reach GP (i.e. the incipient clot formation time), (iii) the clot elasticity at the gel point ($G'_{GP}$), which is a measure of clot strength, and (iv) the fibrin mass, which is a further indicator of the arrangement and extent of the fibrin network forming the clot microstructure (WO 2006/111758; Evans PA, et al., 2010, Blood, Vol 116(17): 3341-3346; Lawrence, MJ, et al., 2014, Br J Haematol., 168: 571-575; Lawrence, MJ, et al., 2014, Thromb Res., Vol 134(2): 488-94; Lawrence MJ, et al., 2015, Atherosclerosis, Vol 240: 402-407. Stanford, SN, et al., 2015, BMC Neurology, 15:35; Davies, NA, et al., 2015, Thromb Haemost., Vol 114(6):1251-9; Davies, GR, et al., 2016, Intensive Care Medicine, Vol 42(12): 1990-1998). Of these parameters, $d_f$ has been validated as an indicator of the mechanical properties of incipient and mature clots and so has been proposed as a functional biomarker of clotting which can be used to provide a "healthy index" for normal clotting (Evans; and Lawrence 2015 *supra*). Clot microstructure density can also be estimated by microscopy techniques of suitable resolution, e.g. scanning electron microscopy (Baradet, T. C.,et al., 1995, Biophysical journal, Vol 68:1551-1560) and confocal laser scanning microscopy (Fan, K., et al, 2015, Journal of Visualised Experiments, Vol 98, e52019), and by measuring clot permeability (Ząbczyk, M. and Undas, A., 2017, Pol Arch Intern Med., Vol 127 (12): 873-881; Wufsus, AR et al., 2013, Biophysical Journal, Vol 104: 1812-1823; Spero, RC et al., 2011, Biophysical Journal, Volume 101: 943-950; and Pieters M, et al., 2012, Journal of Thrombosis and Haemostasis, Vol 10: 2179-2181).

Based on such studies, it is furthermore becoming clear that the microstructure of blood clots can vary significantly between subjects, and in particular between cohorts of subjects, including between those who have undergone a thromboembolic event, or who are diseased, and healthy subjects. Notably, it has been reported that the fractal dimension, $d_f$, of incipient clots may vary between individuals or cohorts of individuals. These differences in microstructure density are also reflected in work showing that clot permeability may vary between individuals or cohorts of individuals. Accordingly, the microstructure of blood clots (whether incipient, maturing or mature) can vary between individuals or cohorts and as such the density and permeability of blood clots (whether incipient, maturing or mature) can vary between individuals or cohorts.

A blood clot of increased density, more specifically a blood clot with a microstructure of increased density, is stronger, more rigid, less flexible, less permeable, less porous and more resistant to degradation (fibrinolysis), and thus may be more likely to give rise to thrombotic complications than a "normal" clot, which may be accommodated, managed and/or eliminated by the body without deleterious effects on the subject. A subject whose blood clots in this way (i.e. who has a predisposition or tendency to form dense clots, or denser clots than normal or abnormally dense clots (i.e. abnormal clots of reduced permeability)) may be said to have a prothrombotic (or hypercoagulation) and hypofibrinolytic fibrin clot phenotype and will be seen to be less responsive to anticoagulation therapy because any thrombotic event which might occur, even minor events, will be more likely to result in detrimental outcomes. Moreover, when a thrombotic complication arises in a subject a blood clot with a microstructure of increased density will be less susceptible to thrombolytic pharmaceutical intervention and so the success of such therapeutic treatments may be reduced.

Recent studies have shown that the blood of subjects with certain disease states can form abnormally dense incipient clots and thus such subjects are at greater risk of detrimental thrombotic events than subjects in which blood clots with a normal density. Subjects at risk of abnormally dense clots (those which may be described as having a prothrombotic (or hypercoagulation) and hypofibrinolytic fibrin clot phenotype) include those with cancer, e.g. lung cancer, especially late stage cancer (Davies, 2015, supra), those with inflammatory disease, e.g. Type I and Type II diabetes (Jörneskog G, et al., 1996, Diabetologia, Vol 39(12):1519-1523; Carr, M., 2001, Journal of Diabetes and Its Complications, Vol 15: 44-54; Fan, supra) and sepsis (Davies, 2016, supra), those with venous thromboembolism (VTE), or who have suffered a previous VTE event (Lawrence, 2014, supra), those with ischemic stroke (Standford, supra), those with myocardial infarction, e.g. ST segment elevation myocardial infarction (Lawrence, 2015, supra), those with coronary artery disease (Mills JD, et al., 2002, Circulation, Vol 106(15):1938-1942) and those with sickle cell disease (Fan, supra).

Other recent studies have shown that the blood of subjects with certain disease states can form abnormal clots with reduced permeability and thus such subjects are at greater risk of detrimental thrombotic events than subjects in which blood clots with a normal permeability (Ząbczyk and Undas, supra). Such subjects may also be described as having prothrombotic (or hypercoagulation) and hypofibrinolytic fibrin clot phenotype and include those with ischemic stroke, advanced coronary artery disease, myocardial infarction, in-stent thrombosis, no-reflow phenomenon following removal of vascular occlusion, chronic obstructive pulmonary disease (COPD), pulmonary embolism, chronic thromboembolic pulmonary hypertension (CTEPH), end-stage renal disease, abdominal aortic aneurysm (AAA), rheumatoid arthritis, deep vein thrombosis, residual vein obstruction, peripheral arterial disease, diabetes mellitus, and cerebral venous sinus thrombosis.

Pharmaceutical interventions are needed which function to reduce the incidence of, or to prevent or inhibit the occurrence of, blood clots which have an abnormally dense microstructure, e.g. by reducing the density of blood clot microstructure, in patients at risk thereof. In other words, there is a need for anticoagulant therapy which is able in particular to treat or prevent, or to reduce, the formation of abnormally dense clots (i.e. abnormal clots of reduced permeability), particularly in subjects who have been identified to be at risk, or increased risk, thereof, for example by reducing the density of the microstructure of the clots which are formed, e.g. by normalising the clot microstructure. The present invention addresses this need.

Alginates are naturally occurring polysaccharides that have been found to have a number of uses, both clinical (e.g. in wound dressings, as drug carriers and in anti-heartburn preparations) and non-clinical (e.g. in food preparation). They are linear polymers of (1-4) linked β-D-mannuronic acid (M) and/or its C-5 epimer α-L-guluronic acid (G). The primary structure of alginates can vary greatly. The M and G residues can be organised as homopolymeric blocks of contiguous M or G residues, as blocks of alternating M and G residues and single M or G residues can be found interspacing these block structures. An alginate molecule can comprise some or all of these structures and such structures might not be uniformly distributed throughout the polymer. In the extreme, there exists a homopolymer of guluronic acid (polyguluronate) or a homopolymer of mannuronic acid (polymannuronate).

Alginates have been isolated from marine brown algae (e.g. certain species of *Durvillea, Lessonia* and *Laminaria*) and bacteria such as *Pseudomonas aeruginosa* and *Azotobacter vinelandii*. Other pseudomonads (e.g. *Pseudomonas fluorescens, Pseudomonas putida,* and *Pseudomonas mendocina*) retain the genetic capacity to produce alginates but in the wild they do not produce detectable levels of alginate. By mutation these non-producing pseudomonads can be induced to produce stably large quantities of alginate.

Alginate is synthesised as polymannuronate and G residues are formed by the action of epimerases (specifically C-5 epimerases) on the M residues in the polymer. In the case of alginates extracted from algae, the G residues are predominantly organised as G blocks because the enzymes involved in alginate biosynthesis in algae preferentially introduce the G neighbouring another G, thus converting stretches of M residues into G-blocks. Elucidation of these biosynthetic systems has allowed the production of alginates with specific primary structures (WO 94/09124, Gimmestad, M et al, Journal of Bacteriology, 2003, Vol 185(12) 3515-3523 and WO 2004/011628).

Alginates are typically isolated from natural sources as large high molecular weight polymers (e.g. an average molecular weight in the range 300,000 to 500,000 Daltons). It is known, however, that such large alginate polymers may be degraded, or broken down, e.g. by chemical or enzymatic hydrolysis to produce alginate structures of lower molecular weight. Alginates that are used industrially typically have an average molecular weight in the range of 100,000 to 300,000 Daltons (such alginates are still considered to be large polymers) although alginates of an average molecular weight of approximately 35,000 Daltons have been used in pharmaceuticals.

More recently alginate oligomers of smaller size (molecular weight) have been proposed for clinical use, most notably to reduce the viscosity of mucus, including in particular hyperviscous mucus such as occurs in sufferers of cystic fibrosis and other respiratory diseases (see WO 2007/039754 and WO 2008/125828) or anti-microbially, against biofilm (WO 2009/068841) and multidrug resistant bacteria (WO 2010/13957). The use of non-sulphated alginate oligomers as general blood anticoagulants has also been proposed in WO 2015/092437. This use is predicated on the basis of a prolongation of overall blood clotting time by alginate oligomers, but a specific effect of reducing the density of the microstructure of a clot, in particular the $d_f$ of an incipient clot, is not disclosed or suggested.

It has now unexpectedly been found that certain alginate oligomers can influence the microstructure of blood clots as they are forming and thereby reduce the density thereof, specifically the density of the fibrin network (microstructure) of the forming clot. This effect is expected to follow through to the mature clot thus resulting in less dense, more permeable, mature clots, specifically mature clots having a less dense microstructure. This effect is reflected in a reduced $d_f$ value at the gel point for clots formed in the presence of such alginate oligomers.

The effects of such alginate oligomers on clot microstructure have also been found to increase the time to gel point ($T_{GP}$), reduce clot strength at the gel point (as measured by $G'_{GP}$) and/or decrease calculated fibrin mass.

The abnormally dense fibrin network formed from glycated fibrinogen by the action of thrombin, which reflects the clotting of diabetic subjects, is also normalised when clotting occurs in the presence of such alginate oligomers.

As a result of these novel findings, such alginate oligomers are now proposed particularly as being for use specifically in subjects predisposed to (at risk of) forming clots which have an abnormally dense microstructure in order to reduce the incidence of, or to prevent or inhibit the occurrence of, such blood clots. Abnormally dense clots and abnormal clots of reduced permeability are more difficult to manage or eliminate by endogenous physiological means or by exogenous thrombolytic interventions and so subjects at risk of such clots are particularly susceptible to detrimental outcomes during thrombotic events. Moreover, anticoagulation therapy of such subjects is less successful overall because the abnormal blood clots to which such subjects are predisposed, should they occur, tend to be resistant, or less responsive to, standard treatments. The treatments of the invention will therefore enhance the anticoagulation therapy of such subjects by reducing the incidence of, or inhibiting (i.e. preventing or reducing) the occurrence of, such abnormal blood clots thereby lowering the risk of detrimental thrombotic events occurring in such subjects and also improving outcomes should such an event take place.

Thus, in a first aspect the invention provides an alginate oligomer for use in the anticoagulation (or antithrombosis) therapy of a human or non-human vertebrate subject at risk of blood clots which have an abnormally dense microstructure, wherein said alginate oligomer has 2 to 40 monomer residues of which at least 80% are guluronic acid (G) residues, and wherein said blood clots which have an abnormally dense microstructure are blood clots which have

(i) a clot permeability Ks value that is at least about 1 x$10^{-9}$·cm$^2$ lower than the Ks value of blood clots of a normal subject from the same species, or
(ii) a $d_f$ of greater than about 1.76 at the gel point.

The above described anticoagulation (or antithrombosis) therapy may comprise treating or preventing a disease or condition associated with blood coagulation (or thrombosis), in particular a disease or condition associated with blood clots which have an abnormally dense microstructure.

In these therapeutic aspects of the invention the alginate oligomer is administered to the subject in need of such treatments (e.g. a subject at risk of thrombosis, or a subject in which thrombosis is occurring or has occurred or is suspected to be occurring or to have occurred (i.e. in which a thrombus is forming or has formed or is suspected to be forming or to have formed)) in an amount which is effective to reduce the incidence of, or to inhibit (i.e. prevent or reduce) the occurrence of, blood clots which have an abnormally dense microstructure. More specifically, the amount of the alginate oligomer administered to the subject will be an amount which is effective to reduce the density and/or increase the permeability of a blood clot which forms (more particularly reduce the density of blood clot microstructure), or which is effective to promote a normal microstructure density in blood clots during the formation thereof (i.e. to normalise microstructure density), in subjects at risk of blood clots which have an abnormally dense microstructure. Such amounts will also be effective to provide anticoagulation therapy or to treat or prevent the target disease or condition associated with blood coagulation and, in particular, the target disease or condition associated with blood clots which have an abnormally dense microstructure. As shown in the Examples, such amounts may also extend the time taken for an incipient clot to reach the gel point, $T_{GP}$, (i.e. the point of haemostatic functionality) and thus directly influence the generation of functional clots and thereby providing a further advantageous aspect to the anticoagulation therapies of the invention.

The skilled person would easily be able to determine what an effective/pharmaceutically effective amount of the alginate oligomer would be on the basis of routine dose response protocols and, conveniently, the routine techniques for assessing blood coagulation and clot structure described herein. The skilled person would, without undue burden, also be able to optimise these amounts and be able to ensure safe use of the alginate oligomers of the invention.

It will be seen, therefore, that in the above described uses the incidence of blood clots which have an abnormally dense microstructure in the subject is reduced, the occurrence of such blood clots in the subject is inhibited (prevented or reduced), the microstructure density of blood clots in the subject is reduced, and/or a normal microstructure density in blood clots during the formation thereof in the subject is promoted. Thus, in certain instances, the alginate oligomer may act, or function, to normalise clot structure (specifically microstructure) and thereby clot permeability. This may facilitate or allow the normal clot dissolution mechanisms of the clot, and may facilitate or enhance therapeutic clot dissolution therapies, as discussed further below. It does not however extend to a direct thrombolytic effect.

In a more specific embodiment the above described anticoagulation (or antithrombosis) therapy may reduce the incidence of, or inhibit the occurrence of, or inhibit the formation of, blood clots which have an abnormally dense microstructure.

In another more specific embodiment, the above described anticoagulation (or antithrombosis) therapy may reduce the density of blood clot microstructure.

In a further more specific embodiment the above described anticoagulation (or antithrombosis) therapy may promote a normal microstructure density in blood clots during the formation thereof.

The above described embodiments may be in the context of the treatment or prevention of a disease or condition associated with blood coagulation (or thrombosis), in particular a disease or condition associated with blood clots which have an abnormally dense microstructure, in the subject.

As a further advantage, the alginate oligomers of use in the invention are able to extend $T_{GP}$ and, as such, may act or function to control directly the point at which a clot becomes haemostatically functional. This facilitates or allows extremely fine control of a subject's blood clotting and thus the most effective, precise and, therefore, safe treatments. Anticoagulation therapies which act at points further up the clotting pathway cannot exert control on the pathway with the same precision as their effects are removed from the final stages of clot formation and may be amplified through the steps which are downstream of the point of action. This makes their use in therapy difficult and potentially dangerous. A more delayed clot formation time caused by an extended $T_{GP}$ is also believed to reduce the risk of dysfunctional (e.g. abnormally dense, overly strong, more rigid and less permeable) clots forming.

Thus, in certain instances, the above described uses also prolong the $T_{GP}$ of the subject's blood.

The terms "administering" and "use" as used in the context of the therapeutic uses of the invention encompasses any means of delivering the alginate oligomer to the subject, more specifically one or more of the parts of the subject at which a thrombotic event is occurring or at which a thrombotic event may be expected or predicted to occur. The alginate oligomer may be provided in a free and/or an immobilised form, e.g. carried on or contained in a surface which when in use may come into contact with the blood of the subject at the target body site. Alginate oligomers may be applied to or incorporated into the material from which the surface is formed or the material may be impregnated with the oligomer, e.g. alginate oligomers may be coated onto a surface of the material, or provided as part of a surface coating. This may occur before, during or after formation of the surface and may be effected through covalent or non-covalent interactions, e.g. ionic or electrostatic interactions, or, expressed more generally, by adsorption, which may be in any manner. In particular, alginate oligomers of suitable monomer composition may be provided on a surface as a, or part of a, cation-induced gel. The alginate oligomers of the invention, when provided at or on the surface of a material render that material more haemocompatible and thus, in certain instances s, may be considered to be a haemocompatible layer or coating.

In still further instances s the alginate oligomer is provided or included in a haemocompatible coating comprising one or more further haemocompatible compounds, e.g. heparin, heparan sulphate, hyaluronan, polyethylene glycol or dextran, or indeed any bioactive agent which has an anticoagulant activity or property or any agent or material which assists in reducing coagulation, e.g. reducing platelet activation and/or binding of blood components (e.g. proteins) required for coagulation. Such a haemocompatible coating may be provided on the surface of e.g. a device or material which is to be contacted with the blood of a subject.

In other instances the alginate oligomer is not used in an immobilised form. Typically in such instances the alginate oligomer is administered in its native form or as part of a composition, e.g. pharmaceutically acceptable formulation.

A subject at risk of, or predisposed to, blood clots which have an abnormally dense microstructure is a subject whose blood clots (coagulates) with a microstructure which is more dense than that of a normal (healthy) subject of the same species. In accordance with the invention, blood clots which have an abnormally dense microstructure are blood clots which have a clot permeability Ks value that is at least about $1 \times 10^{-9} \cdot cm^2$ lower than the Ks value of blood clots of a normal subject from the same species, or a $d_f$ of greater than about 1.76 at the gel point.

This may include subjects which have higher than normal levels of fibrinogen, e.g. diabetic subjects. A normal level of fibrinogen may be considered to be about 2.5 mg/ml (e.g. 2.2 to 2.7 mg/ml or 2.3 to 2.6 mg/ml). Other subjects may additionally or alternatively form glycated clots as a result of the presence of glycated fibrinogen, e.g. diabetic subjects where circulating levels of glucose are elevated as compared to normal.

As discussed in more detail below, such a subject may be a subject who or which has suffered, or is suffering, a thromboembolic (or, alternatively expressed, thrombotic) event or condition, including in particular such a subject who or which is still forming clots with abnormally dense microstructure despite receiving, or having received, anticoagulant therapy (that is standard anti-coagulant therapy, or therapy with an anticoagulant other than an alginate oligomer), and/or who has not responded, or is not responding, to a clot dissolution therapy, e.g. with a thrombolytic agent. However, in other instances the subject does not have a thromboembolism, more specifically a thromboembolism that is a candidate for a thrombolytic therapy.

In other embodiments, the subject may be suffering from a condition which predisposes to the formation of clots with abnormally dense microstructure, e.g. cancer, type 1 or type 2 diabetes, or a cardiovascular disease or condition or sickle cell disease.

Still further, the subject may be a subject who is undergoing or about to undergo surgery, or implantation or introduction of an at least partially in-dwelling device, or a blood dialysis or apheresis (e.g. plasmapheresis) procedure etc. (i.e. irrespective of past history of a thromboembolic event, or predisposing condition), and who has been determined to form clots with abnormally dense microstructure, or to be at risk of, or predisposed to, forming clots with abnormally dense microstructure.

As noted above, a subset of subjects in a population will have a tendency to form clots with abnormally dense microstructure, or to be at risk thereof, or predisposed thereto, and the present invention is aimed at the therapy of such a subset of subjects.

The microstructure of a clot is the network of entangled, branching fibrin fibres which first provides haemostatic function to the incipient clot and in which platelets and other blood cells are entrapped as the clot matures. The density of a clot's microstructure is determined by the diameter of the fibrin fibres and/or the degree of branching (cross-linking). Thinner fibres and/or greater branching within the fibrin network results in a denser clot microstructure which in turn is stronger and less permeable. Conversely, thicker fibres and/or less branching results in a less dense clot microstructure which in turn is not as strong and more permeable. Fibre length might also play a role with shorter fibres generally giving rise to clots with increased density and longer fibres generally giving rise to clots with increased density.

The microstructure of a clot dictates the overall structure of a clot and so descriptors referring to a clot's microstructure can be taken as equivalent descriptors of a clot's overall structure. Thus, a clot with dense microstructure can be considered a clot with a dense structure overall. In addition, the properties of microstructure seen in incipient clots, e.g. at the gel point as shown by the rheological techniques of use in the present invention, are observed in mature clots (Lawrence, 2015, supra). As such observations made or properties assigned to an incipient clot may be extrapolated to mature or maturing forms of that clot.

Clot density may be assessed visually, with or without computer assistance, using a microscopy technique of suitable resolution. Scanning electron microscopy and confocal laser scanning microscopy are commonly used (e.g. Baradet, supra; and Fan, supra). Abnormal density may be assessed by comparison of test clots, specifically the diameter and degree of branching of the fibrin fibres therein, with controls from healthy subjects.

More conveniently, and more accurately, clot density (specifically the density of clot microstructure) may be assessed via the rheological analysis of incipient clots formed from whole blood at the gel point of the clot, e.g. as described in WO 2006/111758 and Evans, Lawrence, 2014, Br J Haematol., Lawrence, 2014, Thromb Res., Lawrence 2015, Stanford, and Davies, all supra). Measurements made in relation to incipient clots formed from the blood of a subject correlate to the density of mature clots in the subject (Lawrence 2015, supra). More specifically, the quantitative parameter fractal dimension ($d_f$), may be calculated from measurements of the viscoelastic properties of clotting whole blood as described in the above references and used to compare the relative density of clot microstructure between test clots and clots from healthy controls. An incipient clot which has a $d_f$ which is greater than that of an incipient clot from a healthy (i.e. normal) control, preferably a cohort of healthy controls, is an incipient clot which has an abnormally dense microstructure and would give rise to a mature clot of abnormally dense microstructure.

The $d_f$ of an incipient clot at its gel point is a highly reproducible parameter and it has been found that the mean $d_f$ of a blood clot formed from unadulterated whole blood from a healthy human subject is equal to about 1.73, e.g. $1.73 \pm 0.03$ (termed a "healthy index" in the art). In accordance with the invention a blood clot with an abnormally dense microstructure is considered to be a clot which, at its gel point, has (had) a $d_f$ (e.g. mean $d_f$) of greater than about 1.76, e.g. a clot which at its gel point has a $d_f$ of at least about 1.765, 1.77, 1.775, 1.78, 1.785, 1.79, 1.795 or at least about 1.8. This may be determined in a sample of untreated ("unadulterated") whole blood from a subject. In particular, $d_f$ may be determined as disclosed in Evans *et al.*, 2010, (supra) or Lawrence *et al.* 2014, Thrombosis Research (supra). In the disclosed methods, the gel point (GP) is detected by measuring the change in phase angle ($\delta$) at a range of different frequencies, with respect to time, and recording the point at which $\tan\delta$ becomes independent of frequency.

More particularly, $d_f$ may be determined by transferring a sample (or an aliquot of a sample), e.g. about 10 ml, of whole blood, preferably directly, and immediately or shortly after sampling, to a TA instruments ARES rheometer and taking sequential frequency and FTMS measurements of G' (dynamic rigidity) and G" (loss modulus) in the linear viscoelastic regime at 37 °C (or another test temperature if desired, e.g. 1°C to 50°C), immediately after sample loading (or attainment of test temperature). G' and G" are the elastic and viscous components respectively of the complex shear modulus $G^*(\omega)$, and at the gel point scale as power laws in frequency, $\omega$, as $G'(\omega) \sim G''(\omega) \sim \omega^\alpha$. GP is identified by attainment of frequency independence of $\tan\delta$ (= G"/G'). $\delta$ represents the phase angle between stress and shear waveforms in small amplitude oscillatory shear measurements and is related to the exponent $\alpha$ as ($\delta = \alpha\pi/2$). The value of $\alpha$ is a sensitive measure of the degree of gel network branching and may be used to calculate $d_f$ by the use of the equation:

$$d_f = (D+2)(2\alpha-D)/(2(\alpha-D), \qquad \text{Formula I}$$

where D is the space dimension (e.g. where D=3).

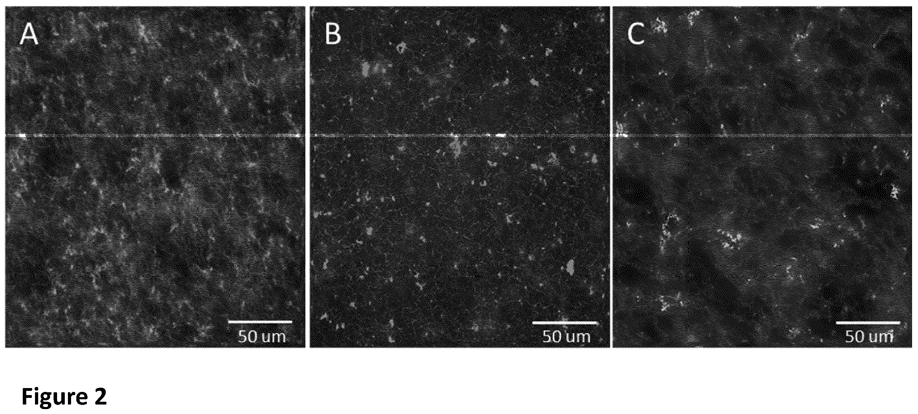

It has been reported in the art that an increased $d_f$ in an incipient clot correlates with a reduced fibre width, greater branching and thus density in the mature clot, and thus that a reduced $d_f$ in an incipient clot is reflected in a change in the structure of the maturing or mature clot; comparisons of SEM micrographs of mature (fully formed) clots with the corresponding $d_f$ values shows that the incipient clot microstructure ($d_f$) mirrors the changes observed in the mature clot, as seen in the SEM images. A change in the clot to thinner fibres and significantly less cross-linking and density corresponds to a decrease in both $d_f$ and $G'_{GP}$ and an increase in $T_{GP}$ (see Lawrence *et al.*, 2015, supra, particularly Figure 2). Thus, there is an established connection between the structure of a mature clot and the microstructure of an incipient clot, with a more permeable, less branched and mechanically weaker mature clot being associated with a reduced $d_f$ at the gel point.

The density of a clot's microstructure may further be assessed indirectly by measuring clot permeability. Clot permeability may, for instance, be measured as described in Ząbczyk and Undas, Wufsus et al., and Pieters M, et al. (supra) following general Formula II

$$Ks\ (\times 10^{-9}\ cm^2) = (Q \times L \times \eta)/(T \times A \times \Delta P) \qquad \text{Formula II}$$

wherein Ks = clot permeability (Darcy constant); Q = volume of liquid (ml) passed through in time T; $\eta$ = viscosity (poise); L = clot length (cm); A = crosssectional area of clot container ($cm^2$); T = time (s); and $\Delta P$ = pressure drop (in dyne $cm^{-2}$).

Clot permeability may, for instance, also be measured as described in Spero et al (supra) by monitoring the diffusion of nanoparticles in the clot and using the effective medium theory. Spero describes a high-throughput assay based on this technique.

Abnormal clot permeability may be assessed by comparison of test clots with controls from healthy subjects, wherein a clot permeability that is significantly reduced as compared to that of one or more clots from healthy subjects is indicative of a clot which has an abnormally dense microstructure. In certain embodiments a Ks value that is at least about $1 \times 10^{-9} \cdot cm^2$ lower, e.g. at least about 1.3, 1.5, 2.0, 2.3, 2.5, 3.0, 3.5, 4.0, 4.5 or $5.0 \times 10^{-9} \cdot cm^2$ lower, than that of a healthy control value is indicative of a clot which has an abnormally dense microstructure.

Thus, a clot which has an abnormally dense microstructure may also be described as a clot with reduced permeability (as compared to clots from a normal or healthy subject), or a permeability which is less than normal, or a clot which is abnormally pervious. These terms may all be used interchangeably.

Clot permeability and by extension density may also described in terms of porosity. An abnormal clot with reduced permeability or a clot with abnormally dense microstructure may have (more specifically may have a microstructure which has) fewer and/or smaller pores than normal. Such clots may be considered abnormally porous, i.e. an abnormal clot with reduced porosity.

A subject whose blood forms a clot which has an abnormally dense microstructure may be considered to be a subject at risk of, or predisposed to, blood clots which have an abnormally dense microstructure.

Subjects which have been identified as being at risk of blood clots which have an abnormally dense microstructure include subjects with cancer, e.g. lung cancer, especially late stage cancer, those with inflammatory disease, e.g. Type I and Type II diabetes and sepsis, those with venous thromboembolism (VTE), or who have suffered a previous VTE event, those with ischemic stroke, those with myocardial infarction, e.g. ST segment elevation myocardial infarction, and those with coronary artery disease. Other such subjects include those with in-stent thrombosis, no-reflow phenomenon following removal of vascular occlusion, COPD, pulmonary embolism, CTEPH, end-stage renal disease, AAA, rheumatoid arthritis, deep vein thrombosis, residual vein obstruction, peripheral arterial disease, and cerebral venous sinus thrombosis. Other such subjects are subjects with sickle cell disease.

Thus, the subject at risk of blood clots which have an abnormally dense microstructure of the invention may be a subject who or which also has one or more of the above diseases or conditions. The treatment of subjects with cancer, e.g. lung cancer, and those with Type I or Type II diabetes in accordance with the invention may be preferred in certain embodiments.

As mentioned already, in other instances the subject does not have a thromboembolism, more specifically a thromboembolism that is a candidate for a thrombolytic therapy.

In other instances, the subject may have been prior-treated with an anticoagulant therapy (other than with an alginate oligomer) and/or clot dissolution therapy. For example, the subject may be suffering from a thromboembolic condition, or may have suffered a previous thromboembolic event. In particular, such a subject may have been non-responsive to the therapy, and/or the blood of the subject may be seen to be capable of forming abnormally dense clots (e.g. clots with an increased $d_f$), despite the previous or on-going therapy.

The assessment of whether or not a subject's blood forms a clot which has an abnormally dense microstructure is typically performed on whole blood which has been isolated from the subject. In certain instances, the isolated whole blood is allowed to clot and the resultant clot is analysed by microscopy to determine its relative density as compared to healthy (normal) controls or has its permeability measured (e.g. as described in the references cited herein) and compared to healthy (normal) controls. In other instances, the isolated whole blood is allowed to clot during rheological analysis as described in the references cited herein and $d_f$ is determined at the gel point as described in the references cited herein, or set out above.

In certain instances, the blood samples used will not contain pharmaceutically effective amounts of anticoagulation, anti-platelet and/or thrombolytic drugs. Thus, in preferred instances, at the time the blood sample is isolated from the subject, the subject will not have pharmaceutically effective levels of anticoagulation, anti-platelet and/or thrombolytic drugs in its circulation.

However, in other instances, the blood sample may be taken from a subject receiving anticoagulant, anti-platelet and/or thrombolytic agents and may accordingly contain such an agent.

In accordance with the invention a subject is considered to be at risk of, or predisposed to, blood clots which have an abnormally dense microstructure if in the absence of pharmaceutically effective amounts of anticoagulation, anti-platelet and/or thrombolytic drugs the blood of the subject has the above described characteristics of blood which forms clots of abnormally dense microstructure, even if the subject subsequently receives an anticoagulation, anti-platelet and/or thrombolytic drug prior to, during or after receiving treatment in accordance with the invention. In another instance, the subject may be at risk of, or predisposed to, blood clots which have an abnormally dense microstructure if in the presence of pharmaceutically effective amounts of anticoagulation, anti-platelet and/or thrombolytic drugs the blood of the subject has the above described characteristics of blood which forms clots of abnormally dense microstructure.

Thus, the therapeutic uses of the invention may also be preceded by a step in which the subject is identified or diagnosed as a subject at risk of, or predisposed to, blood clots which have an abnormally dense microstructure, e.g. as described above.

In other instances the therapeutic uses of the invention may be preceded by a step in which the subject is identified or diagnosed as a subject at risk of thrombosis, or a subject in which a thrombus is forming or has formed or is suspected to be forming or to have formed, e.g. a subject having, suspected to have, or at risk of a disease or condition associated with blood coagulation. The $T_{GP}$ of the subject's blood may also be measured.

In other instances the therapeutic uses of the invention may be followed by a step in which the blood of the subject is tested to assess whether or not it forms a clot which has an abnormally dense microstructure and/or to measure the microstructure density of clots formed therefrom, e.g. as described above.

In other instances the therapeutic uses of the invention may be followed by a step in which the subject is monitored for an effect on its risk of thrombosis, the thrombotic event in question and/or a change in condition or clinical status associated with blood coagulation. For example, the subject is monitored for the formation of a blood clot, or number of clots, and/or an existing blood clot is monitored for changes in size, shape and/or integrity, e.g. using the techniques detailed below. In other instances the additional step may be a step in which the subject is monitored for onset of a disease or condition associated with blood coagulation or changes in a pre-existing disease or condition associated with blood coagulation or its risk of a detrimental outcome associated with a disease or condition associated with blood coagulation. In other instances the additional step may be a step in which the subject's blood is tested to assess its capacity to coagulate, e.g. using the coagulation tests detailed below. The $T_{GP}$ of the subject's blood may also be measured.

The results obtained may be compared to measurements taken before treatment in accordance with the invention was commenced and/or during treatment.

The incidence of blood clots which have an abnormally dense microstructure in a subject is a reference to the number of such clots in the subject, or a part thereof, over a unit of time, i.e. the frequency at which such clots may be detected in the subject. A reduction in incidence refers to a lowering in the number of such clots in the subject, or part thereof, over a unit of time, i.e. a reduction in the frequency at which such clots may be detected in the subject. In accordance with the invention treatment with the alginate oligomer will reduce the incidence of blood clots which have an abnormally dense microstructure as compared to the incidence of such clots prior to treatment. Expressed numerically, this may be a reduction in the incidence of such clots of at least about 5%, e.g. at least about 10%, 15%, 20%, 25%, 30%, 35%, 40%, 45%, 50%, 55%, 60%, 65%, 70%, 75%, 80%, 85%, 90% or about 95%, as compared to the incidence of such clots prior to treatment.

The inhibition of the occurrence of blood clots which have an abnormally dense microstructure in a subject is a reference to any negative effect on the number of blood clots which have an abnormally dense microstructure in a subject, or a part thereof, at any particular time. Encompassed therefore is the absolute prevention of blood clots which have an abnormally dense microstructure, but also a reduction in or limitation to the numbers of such clots in the subject, or part thereof, at any particular time. In accordance with the invention, treatment with thealginate oligomer will inhibit the occurrence of blood clots which have an abnormally dense microstructure as compared to the occurrence of such clots prior to, or in the absence of, treatment. Expressed numerically, the inhibition of the occurrence of such clots may be considered to be a reduction in the occurrence of such clots of at least about 5%, e.g. at least about 10%, 15%, 20%, 25%, 30%, 35%, 40%, 45%, 50%, 55%, 60%, 65%, 70%, 75%, 80%, 85%, 90% or about 95%, as compared to the occurrence of such clots prior to treatment. Prevention of the occurrence of such clots is a 100% reduction in the occurrence of such clots as compared to the occurrence of such clots prior to treatment.

A reduction in the density of clot microstructure in a subject may be considered to be any effect on the clotting blood of the subject which results in a looser or more permeable or more porous fibrin network structure as compared to the fibrin network structure of blood clots formed from the blood of the subject in the absence of thealginate oligomer, e.g. which results in the diameter and/or the length of the fibrin fibres therein and/or the extent of branching and/or cross-linking of said fibrin fibres being reduced and/or the number of fibres per unit area being increased. This may be either in incipient, maturing or mature clots. References to blood clots of reduced microstructure density or blood clots which have a less dense microstructure may be interpreted analogously. Microstructure density and any differences therein may be estimated by visual inspection, with or without computer assistance, of microscopy images of clots formed from the blood of the subject before and after the treatments of the invention. Preferably density is reduced by at least about 2%, e.g. at least about 5%, 10%, 15% or about 20%. More quantitatively, a reduced density of blood clot microstructure in a subject following treatment may be expressed as a value of $d_f$ which is lower than a corresponding value obtained for a blood clot formed prior to treatment. This may be reduction of at least about 0.005, e.g. at least about 0.01, 0.015, 0.02, 0.025, 0.03, 0.35, 0.04, 0.045, 0.05, 0.06, 0.07, 0.08, 0.09, or about 0.1. In preferred embodiments the $d_f$ of clots formed from a subject's blood is reduced by the treatments of the invention to less than or equal to about 1.73 (e.g. to less than or equal to 1.73 $\pm$ 0.03, or less than or equal to about 1.70, 1.705, 1.71, 1.715, 1.72, 1.725, 1.73, 1.735, 1.74, 1.745, 1.75, 1.755 to about 1.76, or about 1.70 to about 1.705, 1.71, 1.715, 1.72, 1.725, 1.73, 1.735, 1.74, 1.745, 1.75, 1.755 or 1.76), i.e. to a $d_f$ of blood clots from healthy subjects (which may be referred to as a normal blood clot microstructure density or the healthy index of blood clotting). In other words, the $d_f$ of clots formed from a subject's blood is normalised.

In other embodiments the Ks value of clots formed from a subject's blood is increased by the treatments of the invention by at least about $1 \times 10^{-9}$ cm$^2$, e.g. at least about 1.3, 1.5, 2.0, 2.3, 2.5, 3.0, 3.5, 4.0, 4.5 or $5.0 \times 10^{-9}$ cm$^2$, and preferably to that of a healthy control value. In other words, the Ks value of clots formed from a subject's blood is normalised.

References to promoting a normal microstructure density in blood clots during the formation thereof in a subject at risk of clots which have an abnormally dense microstructure are references to any effect on the clotting blood of the subject which results in the formation of blood clots which have a microstructure density which is less dense than (or reduced compared to) blood clots formed in the subject's blood prior to treatment. Thus, the clots which form may more closely resemble normal clots, or clots formed from blood with a "healthy index" etc. This may therefore be regarded as normalisation of the clots (more particularly normalisation of clot structure), e.g. in terms of their density and/or permeability. The reference may also refer to increases in the proportion of blood clots of reduced microstructure density amongst the population of blood clots in the subject, e.g. at a particular time or over a unit time. Definitions of less dense or reduced density provided above apply here. In certain instances it is a reference to any effect on the clotting blood of the subject which results in the formation of blood clots which have a normal microstructure density or which increases the proportion thereof in the population of blood clots in the subject, e.g. at a particular time or over a unit time. Definitions of normal blood clot microstructure density provided above apply here.

As noted above, alginates typically occur as polymers of an average molecular mass of at least 35,000 Daltons, i.e. approximately 175 to approximately 190 monomer residues, although typically much higher and an alginate oligomer may be defined generally as a material obtained by fractionation (i.e. size reduction) of an alginate polymer, commonly a naturally occurring alginate. An alginate oligomer can, generally, be considered to be an alginate of an average molecular weight of less than 35,000 Daltons (i.e. less than approximately 190 or less than approximately 175 monomer residues), in particular an alginate of an average molecular weight of less than 30,000 Daltons (i.e. less than approximately 175 or less than approximately 150 monomer residues) more particularly an average molecular weight of less than 25,000 or 20,000 Daltons (i.e. less than approximately 135 or 125 monomer residues or less than approximately 110 or 100 monomer residues).

Viewed alternatively, an oligomer generally comprises 2 or more units or residues.

More specifically, the alginate oligomers for use according to the invention will contain 2, 3, 4, 5 or 6 to 40, 2, 3, 4, 5 or 6 to 35 or 2, 3, 4, 5 or 6 to 30 monomer residues. Thus, an alginate oligomer for use according to the invention will typically have an average molecular weight of 350, 550, 700, 900 or 1000 to 8000 Daltons, 350, 550, 700, 900 or 1000 to 7000 Daltons, or 350, 550, 700, 900 or 1000 to 6,000 Daltons.

Alternatively put, the alginate oligomer may have a degree of polymerisation (DP), or a number average degree of polymerisation (DPn) of 2 to 40, more preferably 2 to 35, 2 to 30, 2 to 28, 2 to 25, 2 to 22, 2 to 20, 2 to 18, 2 to 17, 2 to 15 or 2 to 12.

Other representative ranges (whether for the number of residues, DP or DPn) include any one of 3, 4, 5, 6, 7, 8, 9, 10 or 11 to any one of 40, 39, 38, 37, 36, 35, 34, 33, 32, 31, 30, 29, 28, 27, 26, 25, 24, 23, 22, 21, 20, 19, 18, 17, 16, 15, 14, 13 or 12.

Other representative ranges (whether for the number of residues, DP or DPn) include any one of 8, 9, 10, 11, 12, 13, 14 or 15 to any one of 40, 39, 38, 37, 36, 35, 34, 33, 32, 31, 30, 29, 28, 27, 26, 25, 24, 23, 22, 21, 20, 19, 18, 17 or 16.

Other representative ranges (whether for the number of residues, DP or DPn) include any one of 11, 12, 13, 14, 15, 16, 17 or 18 to any one of 40, 39, 38, 37, 36, 35, 34, 33, 32, 31, 30, 29, 28, 27, 26, 25, 24, 23, 22, 21, 20 or 19.

In general terms, an alginate oligomer will, as noted above, contain (or comprise) guluronate or guluronic acid (G) and/or mannuronate or mannuronic acid (M) residues or units. An alginate oligomer according to the invention will preferably be composed solely, or substantially solely (i.e. consist essentially of) uronate/uronic acid residues, more particularly solely or substantially solely of G and M residues or G residues, so long as at least 80% of the monomer residues are G residues. Alternatively expressed, in the alginate oligomer of use in the present invention, at least 80%, more particularly at least 85, 90, 95 or 99% of the monomer residues may be uronate/uronic acid residues, or, more particularly G and M residues or G residues, so long as at least 80% of the monomer residues are G residues. In other words, preferably the alginate oligomer will not comprise other residues or units (e.g. other saccharide residues, or more particularly other uronic acid/uronate residues).

The alginate oligomer is preferably a linear oligomer.

Preferably at least 85, 90, 91, 92, 93, 94, 95, 96, 97, 98 or 99% of the monomer residues of the alginate oligomer are guluronate. In one embodiment the alginate oligomer may be an oligoguluronate (i.e. a homooligomer of G, or 100% G)

In a further preferred embodiment, the above described alginates of use in the invention have a primary structure wherein the majority of the G residues are in so called G-blocks. Preferably at least 50%, more preferably at least 70 or 75%, and most preferably at least 80, 85, 90, 92 or 95% of the G residues are in G-blocks. A G block is a contiguous sequence of at least two G residues, preferably at least 3 contiguous G residues, more preferably at least 4 or 5 contiguous G residues, most preferably at least 7 contiguous G residues.

In particular at least 90% of the G residues are linked 1-4 to another G residue. More particularly at least 95%, more preferably at least 98%, and most preferably at least 99% of the G residues of the alginate are linked 1-4 to another G residue.

The alginate oligomer of use in the invention is preferably a 3- to 35-mer, more preferably a 3- to 28-mer, in particular a 4- to 25-mer, e.g. a 5- to 20-mer, especially a 6- to 22-mer, in particular an 8- to 20-mer, especially a 10- to 15-mer, e.g. having a molecular weight in the range 350 to 6400 Daltons or 350 to 6000 Daltons, preferably 550 to 5500 Daltons, preferably 750 to 5000 Daltons, and especially 750 to 4500 Daltons or 2000 to 3000 Daltons or 900 to 3500 Daltons. Other representative alginate oligomers include, as mentioned above, oligomers with 5, 6, 7, 8, 9, 10, 11, 12 or 13 to 40, 35, 28, 25, 22 or 20 residues.

It may be a single compound or it may be a mixture of compounds, e.g. of a range of degrees of polymerization. As noted above, the monomeric residues in the alginate oligomer, may be the same or different and not all need carry electrically charged groups although it is preferred that the majority (e.g. at least 60%, preferably at least 80% more preferably at least 90%) do. It is preferred that a substantial majority, e.g. at least 80%, more preferably at least 90% of the charged groups have the same polarity. In the alginate oligomer, the ratio of hydroxyl groups to charged groups is preferably at least 2:1, more especially at least 3:1.

The alginate oligomer of use in the invention may have a degree of polymerisation (DP), or a number average degree of polymerisation ($DP_n$), of 3-28, 4-25, 6-22, 8-20 or 10-15, or 5-18 or 7-15 or 8-12, especially 10.

The alginate oligomer of use in the invention may have a degree of polymerisation (DP), or a number average degree of polymerisation ($DP_n$), of 3-24, 4-23, 5-22, 6-21, 7-20, 8-19, 9-18, 10-17, 11-16, 12-15 or 13-14 (e.g. 13 or 14).

The alginate oligomer of use in the invention may have a degree of polymerisation (DP), or a number average degree of polymerisation (DPn), of 4-25, 5-24, 6-23, 7-22, 8-21, 9-20, 10-19, 11-18, 12-17, 13-16, 14-15 (e.g. 14 or 15).

The alginate oligomer of use in the invention may have a degree of polymerisation (DP), or a number average degree of polymerisation ($DP_n$), of 5-26, 6-25, 7-24, 8-23, 9-22, 10-21, 11-20, 12-19, 13-18, 14-17 or 15-16 (e.g. 15 or 16).

The alginate oligomer of use in the invention may have a degree of polymerisation (DP), or a number average degree of polymerisation ($DP_n$), of 4-40, 4-35, 4-30, 4-28, 4-26, 4-22, 4-20, 4-18, 4-16 or 4-14.

The alginate oligomer of use in the invention may have a degree of polymerisation (DP), or a number average degree of polymerisation ($DP_n$), of 5-40, 5-25, 5-22, 5-20, 5-18, 5-23, 5-20, 5-18, 5-16 or 5-14.

The alginate oligomer of use in the invention may have a degree of polymerisation (DP), or a number average degree of polymerisation ($DP_n$), of 6-40, 6-35, 6-30, 6-28, 6-26, 6-24, 6-20, 6-19, 6-18, 6-16 or 6-14.

The alginate oligomer of use in the invention may have a degree of polymerisation (DP), or a number average degree of polymerisation ($DP_n$), of 8-40, 8-35, 8-30, 8-28, 8-25, 8-22, 8-20, 8-18, 8-16 or 8-14.

The alginate oligomer of use in the invention may have a degree of polymerisation (DP), or a number average degree of polymerisation ($DP_n$), of 9-40, 9-35, 9-30, 9-28, 9-25, 9-22, 9-20, 9-18, 9-16 or 9-14.

The alginate oligomer of use in the invention may have a degree of polymerisation (DP), or a number average degree of polymerisation ($DP_n$), of 10-40, 10-35, 10-30, 10-28, 10-25, 10-22, 10-20, 10-18, 10-16 or 10-14.

The alginate oligomer of use in the invention may have a degree of polymerisation (DP), or a number average degree of polymerisation ($DP_n$), of 11-40, 11-35, 11-30, 11-28, 11-25, 11-22, 11-20, 11-18, 11-16 or 11-14.

The alginate oligomer of use in the invention may have a degree of polymerisation (DP), or a number average degree of polymerisation ($DP_n$), of 12-40, 12-35, 12-30, 12-28, 12-25, 12-22, 12-20, 12-18, 12-16 or 12-14.

The alginate oligomer of use in the invention may have a degree of polymerisation (DP), or a number average degree of polymerisation ($DP_n$), of 13-40, 13-35, 13-30, 13-28, 13-25, 13-22, 13-20, 13-18, 13-16 or 13-14.

The alginate oligomer of use in the invention may have a degree of polymerisation (DP), or a number average degree of polymerisation ($DP_n$), of 14-40, 14-35, 14-30, 14-28, 14-25, 14-22, 14-20, 14-18, 14-16 or 14-15.

The alginate oligomer of use in the invention may have a degree of polymerisation (DP), or a number average degree of polymerisation ($DP_n$), of 15-40, 15-35, 15-30, 15-28, 15-25, 15-22, 15-20, 15-18 or 15-16.

The alginate oligomer of use in the invention may have a degree of polymerisation (DP), or a number average degree of polymerisation ($DP_n$), of 18-40, 18-35, 18-30, 18-28, 18-25, 18-22 or 18-20.

Preferably the alginate oligomer of use in the invention is substantially free, preferably essentially free, of alginate oligomers having a degree of polymerisation outside of the ranges disclosed herein. This may be expressed in terms of the molecular weight distribution of the alginate oligomer of use in the invention, e.g. the percentage of each mole of the alginate oligomer being used in accordance with the invention which has a DP outside the relevant range. The molecular weight distribution is preferably such that no more than 10%, preferably no more than 9, 8, 7, 6, 5, 4, 3, 2, or 1% mole has a DP of three, two or one higher than the relevant upper limit for $DP_n$. Likewise it is preferred that no more than 10%, preferably no more than 9, 8, 7, 6, 5, 4, 3, 2, or 1% mole has a DP below a number three, two or one smaller than the relevant lower limit for $DP_n$.

Suitable alginate oligomers are described in WO2007/039754, WO2007/039760, WO 2008/125828, and WO2009/068841.

Representative suitable alginate oligomers have a $DP_n$ in the range 5 to 30, a guluronate/galacturonate fraction ($F_G$) of at least 0.80, a mannuronate fraction ($F_M$) of no more than 0.20, and at least 95 mole% of DP no more than 25.

Further suitable alginate oligomers have a number average degree of polymerization in the range 7 to 15 (preferably 8 to 12), a guluronate/galacturonate fraction ($F_G$) of at least 0.85 (preferably at least 0.90), a mannuronate fraction ($F_M$) of no more than 0.15 (preferably no more than 0.10), and having at least 95% mole with a degree of polymerization less than 17 (preferably less than 14).

Further suitable alginate oligomers have a number average degree of polymerization in the range 5 to 18 (especially 7 to 15), a guluronate/galacturonate fraction ($F_G$) of at least 0.80 (preferably at least 0.85, especially at least 0.92), a mannuronate fraction ($F_M$) of no more than 0.20 (preferably no more than 0.15, especially no more than 0.08), and having at least 95% mole with a degree of polymerization less than 20 (preferably less than 17).

Further suitable alginate oligomers have a number average degree of polymerization in the range 5 to 18, a guluronate/galacturonate fraction ($F_G$) of at least 0.92, a mannuronate fraction ($F_M$) of no more than 0.08, and having at least 95% mole with a degree of polymerization less than 20.

Further suitable alginate oligomers have a number average degree of polymerization in the range 5 to 18 (preferably 7 to 15, more preferably 8 to 12, especially about 10), a guluronate/galacturonate fraction ($F_G$) of at least 0.80 (preferably at least 0.85, more preferably at least 0.90, especially at least 0.92, most especially at least 0.95), a mannuronate fraction ($F_M$) of no more than 0.20 (preferably no more than 0.15, more preferably no more than 0.10, especially no more than 0.08, most especially no more than 0.05), and having at least 95% mole with a degree of polymerization less than 20 (preferably less than 17, more preferably less than 14).

Further suitable alginate oligomers have a number average degree of polymerization in the range 7 to 15 (preferably 8 to 12), a guluronate/galacturonate fraction ($F_G$) of at least 0.92 (preferably at least 0.95), a mannuronate fraction ($F_M$) of no more than 0.08 (preferably no more than 0.05), and having at least 95% mole with a degree of polymerization less than 17 (preferably less than 14).

Further suitable alginate oligomers have a number average degree of polymerization in the range 5 to 18, a guluronate/galacturonate fraction ($F_G$) of at least 0.80, a mannuronate fraction ($F_M$) of no more than 0.20, and having at least 95% mole with a degree of polymerization less than 20.

Further suitable alginate oligomers have a number average degree of polymerization in the range 7 to 15, a guluronate/galacturonate fraction ($F_G$) of at least 0.85, a mannuronate fraction ($F_M$) of no more than 0.15, and having at least 95% mole with a degree of polymerization less than 17.

Further suitable alginate oligomers have a number average degree of polymerization in the range 7 to 15, a guluronate/galacturonate fraction ($F_G$) of at least 0.92, a mannuronate fraction ($F_M$) of no more than 0.08, and having at least 95% mole with a degree of polymerization less than 17.

Further suitable alginate oligomers have a number average degree of polymerization in the range 5 to 20, a guluronate fraction ($F_G$) of at least 0.85 and a mannuronate fraction ($F_M$) of no more than 0.15.

In a still further embodiment, the alginate oligomers of use in the invention comprise a sequence of alternating M and G residues. A sequence of at least three, preferably at least four, alternating M and G residues represents an MG block. Preferably the alginate oligomers of use in the invention comprise an MG block. Expressed more specifically, an MG block is a sequence of at least three contiguous residues consisting of G and M residues and wherein each non-terminal (internal) G residue in the contiguous sequence is linked 1-4 and 4-1 to an M residue and each non-terminal (internal) M residue in the contiguous sequence is linked 1-4 and 4-1 to a G residue. Preferably the MG block is at least 5 or 6 contiguous residues, more preferably at least 7 or 8 contiguous residues.

In a further embodiment the minority uronate in the alginate oligomer (i.e. mannuronate or guluronate) is found predominantly in MG blocks. In this embodiment preferably at least 50%, more preferably at least 70 or 75% and most preferably at least 80, 85, 90 or 95% of the minority uronate monomers in the MG block alginate oligomer are present in MG blocks. In certain instances the terminal uronic acid residues of the oligomers of use in the invention do not have a double bond, especially a double bond situated between the $C_4$ and $C_5$ atom. Such oligomers may be described as having saturated terminal uronic acid residues. Thus, in other instances the alginate oligomers of use in the invention have unsaturated terminal uronic acid residues. The skilled man would be able to prepare oligomers with saturated terminal uronic acid residues without undue burden. This may be through the use of production techniques which yield such oligomers, or by converting (saturating) oligomers produced by processes that yield oligomers with unsaturated terminal uronic acid residues.

The alginate oligomer will typically carry a charge and so counter ions for the alginate oligomer may be any physiologically tolerable ion, especially those commonly used for charged drug substances, e.g. sodium, potassium, ammonium, chloride, mesylate, meglumine, etc. Ions which promote alginate gelation e.g. group 2 metal ions may also be used.

While the alginate oligomer may be a synthetic material generated from the polymerisation of appropriate numbers of guluronate and mannuronate residues, the alginate oligomers of use in the invention may conveniently be obtained, produced or derived from natural sources such as those mentioned above, namely natural alginate source materials.

Polysaccharide to oligosaccharide cleavage to produce the alginate oligomer useable according to the present invention may be performed using conventional polysaccharide lysis techniques such as enzymatic digestion and acid hydrolysis. In one favoured instance acid hydrolysis is used to prepare the alginate oligomers of use in the invention. In other instances enzymic digestion is used with an additional processing step(s) to saturate the terminal uronic acids in the oligomers.

Oligomers may then be separated from the polysaccharide breakdown products chromatographically using an ion exchange resin or by fractionated precipitation or solubilisation or filtration. US 6,121,441 and WO 2008/125828 describe a process suitable for preparing the alginate oligomers of use in the invention. Further information and discussion can be found in for example in "Handbooks of Hydrocolloids", Ed. Phillips and Williams, CRC, Boca Raton, Florida, USA, 2000.

The alginate oligomers may also be chemically modified, including but not limited to modification to add charged groups (such as carboxylated or carboxymethylated glycans) and alginate oligomers modified to alter flexibility (e.g. by periodate oxidation).

Alginate oligomers (for example oligoguluronic acids) suitable for use according to the invention may conveniently be produced by acid hydrolysis of alginic acid from, but not limited to, *Laminaria hyperbora* and *Lessonia nigrescens,* dissolution at neutral pH, addition of mineral acid reduce the pH to 3.4 to precipitate the alginate oligomer (oligoguluronic acid), washing with weak acid, resuspension at neutral pH and freeze drying.

The alginates for production of alginate oligomers of use in the invention can also be obtained directly from suitable bacterial sources e.g. *Pseudomonas aeruginosa* or *Azotobacter vinelandii.*

In instances where alginate oligomers which have primary structures in which the majority of the G residues are arranged in G-blocks rather than as single residues are required, algal sources are expected to be most suitable on account of the fact that the alginates produced in these organisms tend to have these structures. The bacterial sources may be more suitable for obtaining alginate oligomers of different structures.

The molecular apparatus involved in alginate biosynthesis in *Pseudomonas fluorescens* and *Azotobacter vinelandii* has been cloned and characterised (WO 94/09124; Ertesvåg, H., et al, Metabolic Engineering, 1999, Vol 1, 262-269; WO 2004/011628; Gimmestad, M., *et al (supra)*; Remminghorst and Rehm, Biotechnology Letters, 2006, Vol 28, 1701-1712; Gimmestad, M. et al, Journal of Bacteriology, 2006, Vol 188(15), 5551-5560) and alginates of tailored primary structures can be readily obtained by manipulating these systems.

The G content of alginates (for example an algal source material) can be increased by epimerisation, for example with mannuronan C-5 epimerases from A. *vinelandii* or other epimerase enzymes. Thus, for example *in vitro* epimerisation may be carried out with isolated epimerases from *Pseudomonas* or *Azotobacter,* e.g. AlgG from *Pseudomonas fluorescens* or *Azotobacter vinelandii* or the AlgE enzymes (AlgE1 to AlgE7) from *Azotobacter vinelandii.* The use of epimerases from other organisms that have the capability of producing alginate, particularly algae, is also specifically contemplated. The *in vitro* epimerisation of low G alginates with *Azotobacter vinelandii* AlgE epimerases is described in detail in Ertesvåg *et al (supra)* and Strugala et al (Gums and Stabilisers for the Food Industry, 2004, 12, The Royal Society of Chemistry, 84 - 94).

To obtain G-block containing alginates or alginate oligomers, epimerisation with one or more *Azotobacter vinelandii* AlgE epimerases other than AlgE4 is preferred as these enzymes are capable of producing G block structures. On the other hand AlgE4 epimerase can be used to create alginates or alginate oligomers with alternating stretches of M/G sequence or primary structures containing single G residue as it has been found that this enzyme seems preferentially to epimerise individual M residues so as to produce single G residues linked to M residues rather than producing G blocks. Particular primary structures can be obtained by using different combinations of these enzymes.

Mutated versions of these enzymes or homologues from other organisms are also specifically contemplated as of use. WO 94/09124 describes recombinant or modified mannuronan C-5 epimerase enzymes (AlgE enzymes) for example encoded by epimerase sequences in which the DNA sequences encoding the different domains or modules of the epimerases have been shuffled or deleted and recombined. Alternatively, mutants of naturally occurring epimerase enzymes, (AlgG or AlgE) may be used, obtained for example by site directed or random mutagenesis of the AlgG or AlgE genes.

A different approach is to create *Pseudomonas* and Azotobacter organisms that are mutated in some or all of their epimerase genes in such a way that those mutants produce alginates of the required structure for subsequent alginate oligomer production, or even alginate oligomers of the required structure and size (or molecular weight). The generation of a number of *Pseudomonas fluorescens* organisms with mutated AlgG genes is described in detail in WO 2004/011628 and Gimmestad, M., *et al,* 2003 *(supra)*. The generation of a number of *Azotobacter vinelandii* organisms with mutated AlgE genes is disclosed in Gimmestad, M., *et al, 2006 (supra).* The skilled man would be able to use this teaching to produce new mutants that could be used to give rise to the alginate oligomers of the invention without undue burden.

A further approach is to delete or inactivate the endogenous epimerase genes from an *Azotobacter or a Pseudomonas* organism and then to introduce one or more exogenous epimerase genes, which may or may not be mutated (i.e. may be wild-type or modified) and the expression of which may be controlled, for example by the use of inducible or other "controllable promoters". By selecting appropriate combinations of genes, alginates of predetermined primary structure can be produced.

A still further approach would be to introduce some or all of the alginate biosynthesis machinery of *Pseudomonas* and/or *Azotobacter* into a non-alginate producing organism (e.g. *E. coli*) and to induce the production of alginate from these genetically modified organisms.

When these culture-based systems are used, the primary structure of the alginate or alginate oligomer products can be influenced by the culture conditions. It is well within the capabilities of the skilled man to adjust culture parameters such as temperature, osmolarity, nutrient levels/sources and atmospheric parameters in order to manipulate the primary structure of the alginates produced by a particular organism.

References to "G residues/G" and "M residues/M" or to guluronic acid or mannuronic acid, or guluronate or mannuronate are to be read interchangeably as references to guluronic acid/guluronate and mannuronic acid/mannuronate (specifically α-L-guluronic acid/guluronate and β-D-mannuronic acid/mannuronate), and further include derivatives thereof in which one or more available side chains or groups have been modified without resulting in a capacity to reduce the incidence of, or prevent or inhibit the occurrence of, blood clots which have an abnormally dense microstructure (more specifically, a capacity to reduce the density of blood clot microstructure, or to promote a normal microstructure density in blood clots during the formation thereof) in subjects at risk of blood clots which have an abnormally dense microstructure that is substantially lower than that of the unmodified oligomer. Common saccharide modifying groups would include acetyl, sulphate, amino, deoxy, alcohol, aldehyde, ketone, ester and anhydro groups. Any of these groups may be used to modify the alginate oligomers according to the present invention. The alginate oligomers may also be chemically modified to add charged groups (such as carboxylated or carboxymethylated glycans), and to alter flexibility (e.g. by periodate oxidation). The skilled man would be aware of still further chemical modifications that can be made to the monosaccharide subunits of oligosaccharides and these can be applied to the alginate oligomers of the invention. In other instances the alginate oligomers are non-sulphated and in other instances the alginate oligomers are unmodified, i.e. consist of unmodified G and/or M residues.

The invention encompasses the use of a single alginate oligomer or a mixture (multiplicity/plurality) of different alginate oligomers. Thus, for example, a combination of different alginate oligomers (e.g. two or more) may be used. In this regard, a combination of alginate oligomers may be selected that together give an advantageous profile of anticoagulation properties. This may be a combination of different oligomer sizes, different G and M contents and/or different monomer arrangements.

As used herein in certain contexts the term "anticoagulation therapy" encompasses not only a reduction in the incidence of, or the prevention or inhibition of the occurrence of, or the normalisation of blood clots which have abnormally dense microstructure, but it further extends to therapeutic interventions which may also prevent or inhibit blood coagulation in general. The term "prevent or inhibit blood coagulation" is therefore used herein to encompass both absolute prevention of blood coagulation and any negative effect on the onset of blood coagulation, on a pre-existing clot, or on the number of clots in the circulating blood or the circulatory system of a subject. For instance, this may be a delay in the initiation of the coagulation process or a slowing in one or more steps of the process or the process as a whole. In certain instances, it specifically includes prolonging $T_{GP}$. This may also be seen in a reduction in the size of a clot, or a reduction in the rate at which a pre-existing or growing clot increases in size. It may further be seen in a reduction in the physical integrity, or any sort of structural deterioration, of a pre-existing or growing clot, although the term does not encompass the direct thrombolysis of, or a direct thrombolytic effect on, an established or pre-existing clot. Nevertheless, the treatments of the invention may still facilitate or allow the normal clot dissolution mechanisms of the clot, and may facilitate or enhance therapeutic clot dissolution therapies, It may further be seen in a reduction in the number of clots in the circulating blood or the circulatory system of the subject, or a reduction in the rate at which the number of clots are increasing. Nevertheless, in accordance with the invention these general effects are in addition to the reduction in the incidence of, or the prevention or inhibition of the occurrence of, or the normalisation of blood clots which have abnormally dense microstructure.

The physical dimensions of a blood clot, in particular mature clots, may be measured by any convenient means, e.g. ultrasound, angiography, CT, or simple visual measurement. In other instances coagulation, and therefore its inhibition or prevention, may be measured by a routine coagulation test (or blood clotting test) conveniently using a blood coagulometer, e.g. thromboelastography, the thrombin generation test, the thrombin time test, the thrombodynamics test, the partial thromboplastin time (PTT) (or activated partial thromboplastin time (aPTT or APTT)) test or the prothrombin time test. Preferably clotting time is measured in terms of $T_{GP}$, which is a sensitive, accurate and direct measure of the kinetics of the formation of a haemostatically functional clot, rather than the less direct and therefore less accurate and less sensitive measures of coagulation mentioned above which are used more routinely.

By "blood coagulation" it is meant the natural process by which an insoluble matrix of fibrin fibres (or filaments) is formed from fluid whole blood or plasma. This includes both the tissue factor pathway and the contact pathway described above. This fibrin matrix and any material entrapped therein are referred to herein as a "blood clot" or a "thrombus" or, more simply, a "clot" The matrix of fibrin fibres of a blood clot is referred to herein as the microstructure of the blood clot.

"Treatment" when used in relation to the treatment of a medical disease or condition associated with blood coagulation in accordance with the invention is used broadly herein to include any therapeutic effect, i.e. any beneficial effect on the disease or condition. Thus, not only included is eradication or elimination of the disease or condition, or cure of the subject of the disease or condition, but also an improvement in the disease or condition or overall well-being of the subject or a reduction in the severity of the disease or the risk of a detrimental outcome associated with the disease or condition for the subject. Thus included, for example, is an improvement in any symptom or sign of the disease or condition, or in any clinically accepted indicator of the disease or condition (for example, a lowering in the number of circulating blood clots or an increase oxygenation downstream of a thrombosis). In the presently claimed treatments it may be that pre-existing blood clots are not fully eradicated or the formation of new clots is not completely halted, but the treatments are sufficient to inhibit these processes to such an extent that the target disease or condition is fully resolved, or at least resolved to some extent, preferably to an extent acceptable to the subject. Treatment thus includes both curative and palliative therapy, e.g. of a pre-existing or diagnosed disease or condition, i.e. a reactionary treatment. However, as mentioned already, the term does not encompass treatment by the direct thrombolysis of, or direct a thrombolytic effect on, an established or pre-existing clot.

"Prevention" when used in relation to the treatment of a medical disease or condition associated with blood coagulation in accordance with the invention is used broadly herein to include any prophylactic or preventative effect. It thus includes delaying, limiting, reducing or preventing the disease or condition or the onset of the disease or condition, or one or more symptoms or indications thereof, or reducing the eventual severity thereof, for example relative to the condition or symptom or indication prior to the prophylactic treatment. Prophylaxis thus explicitly includes both absolute prevention of occurrence or development of the disease or condition, or symptom or indication thereof, and any delay in the onset or development of the condition or symptom or indication, or reduction or limitation of the development or progression or eventual severity of the condition or symptom or indication.

In accordance with the invention, "anticoagulation therapy" and "antithrombotic (or antithrombosis) therapy" include both preventative treatment and therapeutic treatment of an existing or ongoing condition and so these terms should be interpreted consistent with the foregoing.

Diseases and conditions associated with blood coagulation, more specifically unwanted or inappropriate blood coagulation, and in particular coagulation which results in blood clots which have an abnormally dense microstructure, include but are not limited to venous thrombosis (e.g. deep vein, portal vein, renal vein, jugular vein and cerebral venous sinus thrombosis), arterial thrombosis (e.g. coronary, carotid and hepatic artery thrombosis), ischemic heart disease, coronary artery disease, atherosclerosis/atherothrombosis, vein graft failure, arterial graft failure, stroke, cardiac (myocardial) infarction, pulmonary embolism, and thrombophilia (also referred to as hypercoagulability or prothrombotic state) and cardiovascular disease in general. The term venous thrombosis is used herein synonymously and interchangeably with venous thromboembolism. Thus the diseases and conditions include particularly any thrombotic or thromboembolic conditions.

Subjects at risk of blood clots which have an abnormally dense microstructure are highly susceptible to such diseases and conditions and such diseases may be further complicated or exacerbated by such blood clots, thus the alginate oligomers of use in the invention may be used to treat or prevent any and all of these diseases and conditions in subjects at risk of blood clots which have an abnormally dense microstructure. Thus, a reference to a disease or condition associated with blood clots which have an abnormally dense microstructure may be considered to include diseases or conditions which are caused by or complicated or exacerbated by blood clots which have an abnormally dense microstructure, e.g. those listed above.

Treatment may involve systemic or local administration of the alginate oligomers.

A further major clinical problem associated with blood coagulation relates to the induction of thromboses by in-dwelling and implantable medical, surgical or prosthetic devices or within machinery and equipment used in blood dialysis and apheresis (e.g. plasmapheresis). Exposure of blood and/or interstitial fluid to the artificial, i.e. abiotic (e.g. plastic, rubber, silicone, metal or glass), surfaces of such products, equipment and machinery promotes the formation of thromboses. It has also been reported that tissue transplants can give rise to these thromboses and may cause problems at the site of the transplanted tissue or remote parts of the circulatory system should those thromboses embolise. Subjects at risk of blood clots which have an abnormally dense microstructure are highly susceptible to such problems and thus the alginate oligomers of use in the invention may be used to treat or prevent such thrombotic events in subjects at risk of blood clots which have an abnormally dense microstructure.

The alginate oligomers of use in the invention can therefore be used to inhibit or prevent the formation of thromboses on such devices or transplant tissues and in subjects undergoing implantation, tissue transplantation, blood dialysis or apheresis.

Implantable devices include any kind of line, including catheters (e.g. central venous and peripheral venous catheters), heart valves, vascular stents, artificial joints, intrauterine devices, pacemakers, tracheostomy tubes, radiotherapy wires and soft tissue implants. Such devices include devices which, when in use may be partly in-dwelling. Transplant tissue may include heart (e.g. heart valve), lung, kidney, liver, pancreas, intestine and corneal tissue, arterial and venous grafts and skin.

Such treatments may comprise the local or systemic administration of the alginate oligomers to a subject at risk of blood clots which have an abnormally dense microstructure in which an implantable device or a tissue transplant has been or will be implanted or which is undergoing or will undergo dialysis or apheresis. In other instances the implantable/in-dwelling device or tissue transplant may be implanted after being provided with (receiving a treatment with), e.g. a coating of, the alginate oligomer of use in the invention on surfaces that may come into contact with blood. In some instances, this does not include stents, e.g. vascular stents. Such treatments may be applied to the implantable surfaces just prior to, or substantially contemporaneously with, implantation, e.g. by spraying the relevant surfaces of the implantable device or the tissue transplant with a liquid formulation comprising the alginate oligomer.

In other instances the implantable or in-dwelling surface(s) of the devices may contain or carry, e.g. may incorporate, or may be impregnated or coated with, the alginate oligomer of use in the invention. In these instances, the alginate oligomers may be applied directly (e.g. coated, immobilised or attached) onto the implantable/in-dwelling surfaces or provided as part of (e.g. entrapped in or attached to) a surface coating which has been applied to the product or device. This may occur before, during or after formation of the implantable surface and immobilisation of the alginate oligomers or the surface coating may be effected through covalent or non-covalent interactions, e.g. ionic or electrostatic interactions. By way of representative example, the alginate oligomers may be provided on a surface as a, or part of a, cation-induced gel. By way of further example, the alginate oligomer may be entrapped in a polymer or coating applied to the surface, or it may be covalently attached to a matrix polymer used to prepare a polymer coating. The polymer coating may be designed to provide for delayed release of the alginate oligomer, for example by incorporating it (e.g. entrapping it) in different polymer layers. Such delayed release may be over different time periods e.g. from days to months. In still further instances the alginate oligomer may be provided or included in a haemocompatible coating comprising one or more further haemocompatible compounds, e.g. heparin, heparan sulphate, hyaluronan, polyethylene glycol or dextran, or indeed any other bioactive agent having anticoagulant activity, or a compound or agent having a passive haemocompatible property, e.g. a haemocompatible polymer or plastic.

A number of different coating technologies for medical devices and such like are known, for example from SurModics, and any such technology may be used. Indeed, as noted above, it is known to provide medical devices and products with haemocompatible coatings to try to reduce coagulation. "Haemocompatible" is defined herein to mean that that the surface/material/product/device/coating or compound etc. in question does not elicit any negative response when placed in contact with blood or with a composition comprising plasma, or that any such negative response is reduced or inhibited e.g. minimised. A negative response may be coagulation, platelet activation etc., and in particular any response that can lead to, or increase, coagulation.

A haemocompatible material or coating may be "active", i.e. it may comprise bioactive agents which inhibit or prevent coagulation, for example by inhibiting thrombin generation (e.g. heparin), or it may be "passive", and may provide an anticoagulant or haemocompatible effect by e.g. shielding the surface from the blood, or reducing its capacity to induce coagulation, for example by reducing protein adsorption to the surface from the blood, and/or platelet activation. A passive coating may be viewed as "disguising" or "camouflaging" the surface so that it is not recognised as foreign in the blood. Various different active and passive haemocompatible coatings are known in the art. The alginate oligomer may be used in combination with such coatings and may be used to impart an active and/or a passive effect.

Thus, the alginate oligomer may be provided as part of, or included in, a haemocompatible coating, and may be used in combination with one or more haemocompatible compounds as discussed above, including both bioactive agents which may have an anticoagulant effect, e.g. heparin, hyaluronan, dextran etc. and polymers and/or plastics which may have a passive haemocompatible effect.

A further example of a haemocompatible compound is a molecule which may release nitric oxide (NO), which is known to prevent platelet activation. NO-releasing polymers have been developed.

In the context of dialysis and apheresis, consumables, equipment and machinery, including disposable conduits receptacles and filters, may contain or carry, e.g. incorporate or be impregnated or provided with, the alginate oligomer or coatings of the same as described above for implantable devices.

A further major clinical problem associated with blood coagulation relates to the induction of thromboses during surgical procedures. Incisions into the tissues of a subject can become loci for thromboses which in turn may embolise and travel to another part of the circulatory system at which they lodge and give rise to the diseases and conditions discussed above. Subjects at risk of blood clots which have an abnormally dense microstructure are highly susceptible to such problems and thus the alginate oligomers of the invention may be used to treat or prevent such thrombotic events in subjects at risk of blood clots which have an abnormally dense microstructure.

The alginate oligomer of use in the invention can therefore be used before, during and/or after surgical procedures to inhibit or prevent the formation of thromboses at sites of surgical incision. Such treatments may comprise the systemic or, more conveniently, the local administration of the alginate oligomer to a subject undergoing, about to undergo or recovering from a surgical procedure.

Specifically, the alginate oligomer of use in the invention can be taken as a prophylactic treatment by subjects at risk of blood clots which have an abnormally dense microstructure, for example to prevent, or at least minimise the risk, of thrombotic disease (e.g. those described above) as a result of the effects of alginate oligomers in reducing the incidence of, or preventing or inhibiting the occurrence of, abnormally dense blood clots.

The subject may be any human or non-human vertebrate subject, but more particularly may be a vertebrate selected from mammals, birds, amphibians, fish and reptiles. The non-human vertebrate may be a livestock or a domestic animal or an animal of commercial value, including laboratory animals or an animal in a zoo or game park. Representative animals therefore include dogs, cats, rabbits, mice, guinea pigs, hamsters, horses, pigs, sheep, goats, cows, chickens, turkeys, guinea fowl, ducks, geese, parrots, budgerigars, pigeons, salmon, trout, cod, haddock, sea bass and carp. Veterinary uses of the invention are thus covered. The subject may be viewed as a patient. Preferably, the subject is a human.

In certain instances, the subject does not have an infection, e.g. a bacterial, fungal, protozoal, algal or parasite infection, displaying pathological indicators. More particularly in certain such instances, the subject does not have a biofilm infection. In other instances embodiments, the subject does not have hyperviscous mucus or is need of reduction in mucus viscosity. In other instances, the subject does not have a thromboembolism, more specifically a thromboembolism that is a candidate for a thrombolytic therapy. In other instances, the subject does not have an implanted stent, e.g. vascular stent.

A "normal" or "healthy" subject is a subject that is not considered to have, have had or be at significant risk of a thrombotic disease or condition. In certain embodiments, a "normal" or "healthy" subject of use in the invention is a subject which is also not considered to have, have had, or be at significant risk of a disease or condition of the (blood) circulatory system. In certain embodiments a "normal" or "healthy" subject of use in the invention is a subject which is substantially free of serious illness or disease or other medical conditions, or at least is a subject that does not have observable or detectable symptoms of any recognised serious illness or disease. In other embodiments, a "normal" or "healthy" subject will be essentially free of all illness or disease or other medical conditions, or at least does not have observable or detectable symptoms of any recognised illness or disease. In certain embodiments, the blood of a "normal" or "healthy" subject has a healthy index, as indicated for example by a $d_f$ of about 1.73 at the gel point.

The alginate oligomer may be used in the uses of the invention in conjunction or combination with a further blood anti-coagulant (hereinafter "further anticoagulant")

In the context of a therapeutic use, such an anticoagulant agent may be any clinically-useful anticoagulant, e.g. the vitamin K antagonists (e.g. warfarin, acenocoumarol, phenprocoumon, atromentin and phenindione), heparin, low molecular weight heparin, synthetic pentasaccharide inhibitors of factor Xa (e.g. fondaparinux and idraparinux, direct factor Xa inhibitors (e.g. rivaroxaban and apixaban), direct thrombin inhibitors (e.g. hirudin, lepirudin, bivalirudin, argatroban, dabigatran, ximelagatran, antithrombin protein, batroxobin and hementin).

The alginate oligomer may be used in the uses of the invention in conjunction or combination with a clot dissolution therapy, such as in particular a thrombolytic agent (also known as fibrinolytic agents). Thrombolytic agents digest or break down pre-extisting blood clots and include tissue plasminogen activator (t-PA), alteplase, reteplase, tenecteplase, anistreplase, streptokinase and urokinase.

In certain instances, the alginate oligomer may enhance or potentiate the effect of a clot dissolution therapy or therapeutic agent (e.g. thrombolytic agent). It is believed in this regard that by promoting a normal clot structure, and preferably thereby normalising clot permeability, the alginate oligomer may facilitate the clot dissolution activity of a clot dissolution therapy, in particular of a thromobolytic (or fibrinolytic) agent.

The alginate oligomer may be used in the uses of the invention in conjunction or combination with an antiplatelet agent. Antiplatelet agents inhibit platelet aggregation and include the irreversible cyclooxygenase inhibitors (e.g. aspirin), adenosine diphosphate (ADP) receptor inhibitors (e.g. clopidogrel, prasugrel, ticagrelor, ticlopidine, phosphodiesterase inhibitors (e.g. cilostazol), glycoprotein IIB/IIIA inhibitors (e.g. abciximab, eptifibatide, tirofiban) adenosine reuptake inhibitors (e.g. dipyridamole), thromboxane inhibitors (e.g. terutroban)

The alginate oligomers proposed for use according to the invention and the further anticoagulant, thrombolytic agent or antiplatelet agent, may for example be administered together, in a single pharmaceutical formulation or composition, or separately (i.e. separate, sequential or simultaneous administration). Thus, the alginate oligomers of the invention and the further anticoagulant, thrombolytic agent or antiplatelet agent may be combined, e.g. in a pharmaceutical kit or as a combined ("combination") product.

Thus, also described herein are products (e.g. a pharmaceutical kit or a combined ("combination") product) or compositions (e.g. a pharmaceutical composition) wherein the product or composition comprises an alginate oligomer as herein defined and a further anticoagulant, thrombolytic agent and/or antiplatelet agent. Such pharmaceutical products and pharmaceutical compositions are preferably adapted for use in the medical methods of the invention.

In particular there is described a product comprising an alginate oligomer as herein defined and a further anticoagulant, thrombolytic agent and/or antiplatelet agent as a combined product for simultaneous, separate or sequential use for:

(i) the anticoagulation (or antithrombosis) therapy of a human or non-human vertebrate subject at risk of blood clots which have an abnormally dense microstructure; or
(ii) treating or preventing a disease or condition associated with blood coagulation (or thrombosis), in particular a disease or condition associated with blood clots which have an abnormally dense microstructure, in a human or non-human vertebrate subject at risk of blood clots which have an abnormally dense microstructure, or
(iii) inhibiting the incidence, occurrence or formation of blood clots which have an abnormally dense microstructure in a human or non-human vertebrate subject at risk of blood clots which have an abnormally dense microstructure; or
(iv) promoting a normal microstructure density in blood clots during the formation thereof in a human or non-human vertebrate subject at risk of blood clots which have an abnormally dense microstructure.

The alginate oligomers of use in the invention may be administered to the subject in any convenient form or by any convenient means, e.g. by topical, oral, enteral or parenteral routes, or by inhalation. Preferably, the alginate will be administered by topical, oral or parenteral routes, or by inhalation. That alginate oligomers may be administered via many different routes is an advantage over currently available anticoagulants.

The skilled man will be able to formulate the alginate oligomers of use in the invention into pharmaceutical compositions that are adapted for these routes of administration according to any of the conventional methods known in the art and widely described in the literature.

Thus, described herein is a pharmaceutical composition for use in any of the above-mentioned uses comprising an alginate oligomer as defined herein together with at least one pharmaceutically acceptable carrier, diluent or excipient. This composition may also comprise other therapeutic agents as described above.

More specifically, the alginate oligomers of use in the invention may be incorporated, optionally together with other active agents, with one or more conventional carriers, diluents and/or excipients, to produce conventional galenic preparations such as tablets, pills, powders (e.g. inhalable powders), lozenges, sachets, cachets, elixirs, suspensions, emulsions, solutions, syrups, aerosols (as a solid or in a liquid medium), sprays (e.g. nasal sprays), compositions for use in nebulisers, ointments, soft and hard gelatine capsules, suppositories, pessaries, sterile injectable solutions, sterile packaged powders, and the like. Sterile injectable compositions are of particular note.

Examples of suitable carriers, excipients, and diluents are lactose, dextrose, sucrose, sorbitol, mannitol, starches, gum acacia, calcium phosphate, inert alginate polymers, tragacanth, gelatine, calcium silicate, microcrystalline cellulose, polyvinylpyrrolidone, cellulose, water syrup, water, water/ethanol, water/ glycol, water/polyethylene, hypertonic salt water, glycol, propylene glycol, methyl cellulose, methylhydroxybenzoates, propyl hydroxybenzoates, talc, magnesium stearate, mineral oil or fatty substances such as hard fat or suitable mixtures thereof. Excipients and diluents of note are mannitol and hypertonic salt water (saline).

The compositions may additionally include lubricating agents, wetting agents, emulsifying agents, suspending agents, preserving agents, sweetening agents, flavouring agents, and the like. Additional therapeutically active agents may be included in the pharmaceutical compositions, as discussed above in relation to combination therapies above.

Parenterally administrable forms, e.g., intravenous solutions, should be sterile and free from physiologically unacceptable agents, and should have low osmolarity to minimize irritation or other adverse effects upon administration and thus solutions should preferably be isotonic or slightly hypertonic, e.g. hypertonic salt water (saline). Suitable vehicles include aqueous vehicles customarily used for administering parenteral solutions such as sterile water for injection, Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975). The solutions can contain preservatives, antimicrobial agents, buffers and antioxidants conventionally used for parenteral solutions, excipients and other additives which are compatible with the biopolymers and which will not interfere with the manufacture, storage or use of products.

For topical administration the alginate oligomer can be incorporated into creams, ointments, gels, transdermal patches and the like. Simple sterile solutions of alginate oligomer or simple sterile liquid compositions comprising alginate oligomer may be especially convenient for use during surgical procedures and for treating implantable devices. The alginate oligomer can also be incorporated into medical dressings, for example wound dressings e.g. woven (e.g. fabric) dressings or non-woven dressings (e.g. gels or dressings with a gel component). The use of alginate polymers in dressings is known, and such dressings, or indeed any dressings, may further incorporate the alginate oligomers of use in the invention.

Further topical systems that are envisaged to be suitable are in situ drug delivery systems, for example gels where solid, semi-solid, amorphous or liquid crystalline gel matrices are formed in situ and which may comprise the alginate oligomer (which may be any alginate oligomer as herein defined). Such matrices can conveniently be designed to control the release of the alginate oligomer from the matrix, e.g. release can be delayed and/or sustained over a chosen period of time. Such systems may form gels only upon contact with biological tissues or fluids. Typically the gels are bioadhesive. Delivery to any body site that can retain or be adapted to retain the pre-gel composition can be targeted by such a delivery technique. Such systems are described in WO 2005/023176.

The relative content of the alginate oligomer in the compositions of the invention can vary depending on the dosage required and the dosage regime being followed and this will depend on the subject to be treated and the location and identity of the thrombus or the likely site(s) of formation. Preferably, the composition will comprise an amount of alginate oligomer the will provide measurable inhibition or prevention of blood coagulation and/or measurable, preferably clinically relevant, prevention or treatment of the target disease or condition associated with blood coagulation.

Preferably the composition or product will comprise sufficient alginate oligomer that upon administration to a subject or application to a location, the local concentration at the target location of the oligomer will be at least 0.001%, 0.002%, 0.005%, 0.007%, 0.01%, 0.02%, 0.05%, 0.1%, 0.2%, 0.3%, 0.4%, 0.5%, 0.6%, 0.7%, 0.8%, 0.9% or 1% (weight by volume). In other instances the local concentration may be at least 2%, 3%, 4%, 5%, 6%, 7% or 8% and most preferably at least 10% (weight by volume). The skilled man would know that the amounts of alginate can be reduced if a multiple dosing regime is followed or increased to minimise the number of administrations or applications.

The compositions and products of the invention will typically comprise 1% to 99%, 5% to 95%, 10% to 90% or 25% to 75% alginate oligomer, allowance being made for other ingredients.

The invention will be further described with reference to the following nonlimiting Examples in which:

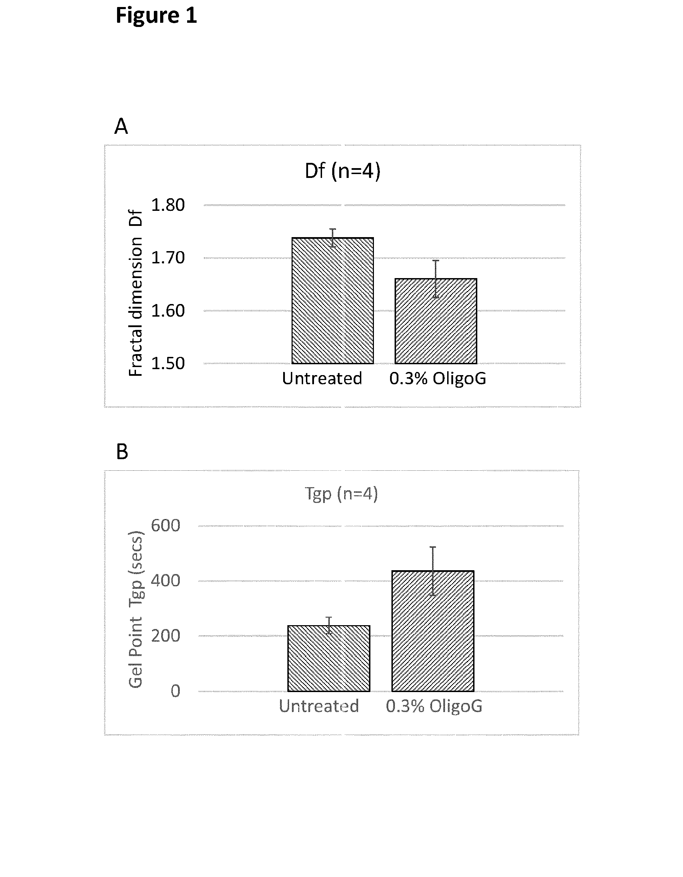

<u>Figure 1</u> presents charts showing the effect of 0.3% (w/v) OligoG on (A) the fractal dimension ($d_f$) and (B) the $T_{GP}$ of blood from 4 healthy subjects, determined as described in Example 1.

<u>Figure 2</u> presents confocal laser scanning microscopy (CLSM) images of normal (A) and glycated fibrin clots (simulated diabetic clots) in the absence (B) or presence of 0.6% OligoG (C).

**EXAMPLES**

Example 1 - determination of $d_f$ and $T_{GP}$ for blood from healthy patients treated with an alginate oligomer

*Materials and methods*

The alginate oligomer tested was OligoG CF-5/20, a G-block alginate oligomer (DP 5 to 20, average molecular weight 3200 Da, 90-95% G residues). OligoG was provided by AlgiPharma AS, Norway.

*Healthy volunteers*

Following informed written consent 10 healthy volunteers were recruited into the study. All participants declared they were not on any medication or had any underlying condition which would affect coagulation either directly or indirectly (5 females and 5 males age range from 23 to 59 years, mean age = 42 years).

*Blood sampling and treatment with alginate oligomer*

Blood was taken atraumatically through a large bore catheter from the antecubital vein (the first 2mls were discarded). OligoG was made up to a 6% solution in saline. For each haemorheological test, a 0.5 ml aliquot from this 6% solution was added to 9.5 ml of whole, unadulterated blood, which was gently inverted several times to ensure thorough mixing (final OligoG concentration - 0.3%). Following mixing, treated blood was immediately placed in an AR-G2 controlled stress rheometer (TA Instruments, UK) fitted with a low inertia doublegap measuring system for analysis and subsequent determination of $d_f$ and $T_{GP}$. Untreated controls were handled analogously. Sample loading from taking the blood to starting the experiment was timed and took less than 60 seconds. Each participant had a baseline control measure.

Specifically, the sample under was loaded into a measuring container mounted on a TA Instruments AR-G2 controlled-stress rheometer (TA Instruments, New Castle, DE, USA) at 37°C ($\pm$ 0.1°C). The system was maintained at the test temperature by means of a peltier temperature control system fitted to a recirculating water bath. In addition, a vapour hood was fitted to minimise any effects due to sample evaporation.

Measurements were begun immediately after sample loading and the evolution of viscoelastic data during coagulation was recorded. An automated numerical method for gel point location was employed to analyse the raw data and the resulting value of $d_f$ and the time taken to reach the Gel Point, $T_{GP}$, were determined.

This involved measurements of the dynamic rigidity (also referred to as shear elastic modulus) (G') and loss modulus (G") components of the complex shear modulus G* and the phase angle between stress and strain waveforms in small amplitude oscillatory shear measurements ($\delta$) over a range of frequencies. Recorded test waveforms were subjected to Fourier analysis with reference to the 3rd harmonic amplitude in order to verify that tests were conducted within the linear viscoelastic range, LVR. The Fourier analysis established the maximum applied strain amplitude required to maintain a linear viscoelastic response at various stages during coagulation. At the Gel Point, G' and G" scale as power-laws in frequency, $\omega$, as $G'(\omega) \sim G''(\omega) \sim \omega^{\alpha}$, the Gel Point being identified by the corresponding frequency independence of the loss tangent, $\tan\delta$ ($= G''/G'$), where $\delta$ is related to the exponent $\alpha$ as $\delta = \alpha\pi/2$. The value of $\alpha$ is a sensitive measure of the degree of gel network branching and was used to calculate $d_f$ using the established relationship $d_f = (D + 2)(2\alpha - D)/2(\alpha - D)$ where D is the space dimension (D = 3 herein).

*Results*

The values obtained for $d_f$ and $T_{GP}$ from 4 patients are shown in Tables 1A and 1B below, and presented graphically in Figure 1. Samples from the remaining 6 patients did not meet stringent inclusion criteria and so were excluded prior to determination of $d_f$ and $T_{GP}$. The higher the value of $d_f$ the more compact is the network, whereas lower values of $d_f$ correspond to more open networks.

Table 1A

|  | $d_f$ | $d_f$ |
| --- | --- | --- |
|  | without OligoG | 0.3% OligoG |
|  | 1.74 | 1.61 |
|  | 1.76 | 1.69 |
|  | 1.72 | 1.67 |
|  | 1.73 | 1.67 |
| Mean | 1.74 | 1.66 |

(continued)

|  | $d_f$ | $d_f$ |
|---|---|---|
|  | without OligoG | 0.3% OligoG |
| SD | 0.02 | 0.03 |
| Ttest | 0.02 |  |

Table 1B

|  | $T_{GP}$ | $T_{GP}$ |
|---|---|---|
|  | Without OligoG | 0.3% OligoG |
|  | 221 | 430 |
|  | 205 | 517 |
|  | 270 | 315 |
|  | 255 | 480 |
| Mean | 238 | 436 |
| SD | 30 | 88 |
| Ttest | 0.04 |  |

As will be apparent from Tables 1A and 1B and the corresponding Figures 1A and 1B, the addition of OligoG to the blood of a healthy subject resulted in a decrease of $d_f$ from a mean value of 1.74 to a mean value of 1.66. The reduced $d_f$ is indicative of a more open, less dense clot microstructure. It is expected that treatment of blood from a patient whose blood clots with abnormal density with OligoG will lower the density of the clots which form and would therefore normalise blood clotting in said subject

$T_{GP}$ is also extended in the OligoG treated samples. Anticoagulation therapeutics which extend $T_{GP}$, i.e. the time in which a haemostatically functional clot is formed, are considered advantageous because such compounds directly control the final stages of the clotting process and therefore will be capable of exerting the most sensitive and accurate control on a patient's blood clotting pathways. Since blood clotting in a subject represents a physiological balance between too little and too much, therapeutics which offer the most sensitivity and the most accuracy in controlling clotting will be those which are the safest and easiest to use.

Example 2 - Confocal laser scanning microscopy analysis of fibrin clot density following alginate oligomer treatment

*Materials and methods*

Fibrin clot structures were formed *in vitro* from human fibrinogen (10% fluorescently labelled), human thrombin, and FXIIIa, and analysed by CLSM according to published methods (Fan et al, supra). By using an artificially glycated form of the human fibrinogen reagent it is possible to simulate diabetic thrombosis conditions, which are known to result in clots with abnormally dense microstructure. These methods were used to examine the structures involved in fibrin clot formation under normal and simulated diabetic thrombosis conditions and observe the effects of OligoG (0.6%) thereon.

*Results*

The results are shown in Figure 2. It is clear from these pictures that CLSM is able to differentiate between clots of normal density and abnormally dense clots. In this instance, the glycated (abnormal) clot (B) is denser with smaller pores than the normal clot (A), and in the presence of 0.6% OligoG this abnormal clot formation is reversed (C). These data show that alginate oligomers are able to normalise the density of blood clots which form from glycated fibrinogen, i.e. in diabetic subjects. It is expected that alginate oligomers would also be able to act similarly to normalise clot density in other subjects which are at risk of blood clots which have an abnormally dense microstructure

**Claims**

1. An alginate oligomer for use in the anticoagulation therapy of a human or non-human vertebrate subject at risk of blood clots which have an abnormally dense microstructure, wherein said alginate oligomer has 2 to 40 monomer residues of which at least 80% are guluronic acid (G) residues, and wherein said blood clots which have an abnormally dense microstructure are blood clots which have

   (i) a clot permeability Ks value that is at least about 1 x10$^{-9}$·cm$^2$ lower than the Ks value of blood clots of a normal subject from the same species, or
   (ii) a d$_f$ of greater than about 1.76 at the gel point.

2. The alginate oligomer for use of claim 1, wherein said anticoagulation therapy comprises treating or preventing a disease or condition associated with blood clots which have an abnormally dense microstructure.

3. The alginate oligomer for use claim 1 or claim 2, wherein the incidence of blood clots which have an abnormally dense microstructure in the subject is reduced and/or the occurrence and/or formation of blood clots which have an abnormally dense microstructure in the subject is inhibited.

4. The alginate oligomer for use of any one of claims 1 to 3, wherein the microstructure density of blood clots in the subject is reduced and/or a normal microstructure density in blood clots during the formation thereof in the subject is promoted.

5. The alginate oligomer for use of claim 2, wherein said disease or condition associated with blood coagulation is cardiovascular disease, ischemic heart disease, coronary artery disease, venous thrombosis, arterial thrombosis, atherosclerosis, atherothrombosis, vein graft failure, arterial graft failure, stroke, cardiac infarction, pulmonary embolism, and thrombophilia

6. The alginate oligomer for use of any one of claims 1 to 5, wherein said blood clots which have an abnormally dense microstructure are blood clots which have

   (i) higher levels of fibrinogen;
   (ii) higher levels of glycated fibrinogen;
   (iii) thinner fibrin fibres;
   (iv) greater fibrin branching;
   (v) shorter fibrin fibres;
   (vi) reduced permeability; and/or
   (vii) reduced porosity,

   as compared to blood clots from a normal subject from the same species.

7. The alginate oligomer for use of any one of claims 1 to 6, wherein the subject is selected from a subject with

   (i) cancer,
   (ii) an inflammatory disease,
   (iii) venous thromboembolism (e.g. pulmonary embolism, deep vein thrombosis, cerebral venous sinus thrombosis),
   (iv) ischemic stroke,
   (v) myocardial infarction,
   (vi) coronary artery disease,
   (vii) in-stent thrombosis,
   (viii) no-reflow phenomenon following removal of vascular occlusion,
   (ix) chronic obstructive pulmonary disease,
   (x) chronic thromboembolic pulmonary hypertension,
   (xi) end-stage renal disease,
   (xii) abdominal aortic aneurysm,
   (xiii) rheumatoid arthritis,
   (xiv) residual vein obstruction,
   (xv) peripheral arterial disease,

(xvi) diabetes mellitus,
(xvii) sepsis, or
(xviii) sickle cell disease.

8. The alginate oligomer for use of any one of claims 1 to 7, wherein the subject

(i) is undergoing, about to undergo or has recently undergone a surgical procedure
(ii) is undergoing or about to undergo implantation of an in-dwelling medical, surgical or prosthetic device or tissue transplant
(iii) has received an in-dwelling medical, surgical or prosthetic device or tissue transplant,
(iv) is undergoing, about to undergo or has recently undergone dialysis and/or apheresis

9. The alginate oligomer for use of any one of claims 1 to 8, wherein the alginate oligomer has a degree of polymerisation (DP), or a number average degree of polymerisation (DPn) of

(i) 2 to 35, 2 to 30, 2 to 25, 2 to 22, 2 to 20, 2 to 18, 2 to 16 or 2 to 14,
(i) 4 to 35, 4 to 30, 4 to 25, 4 to 22, 4 to 20, 4 to 18, 4 to 16 or 4 to 14,
(iii) 6 to 35, 6 to 30, 6 to 25, 6 to 22, 6 to 20, 6 to 18, 6 to 16 or 6 to 14, or
(iv) 8 to 35, 8 to 30, 8 to 25, 10 to 25, 10 to 22, 10 to 20, 10 to 18, or 10 to 14.

10. The alginate oligomer for use of claims 1 to 10, wherein the alginate oligomer is a 2- to 35-mer, 2- to 30-mer, 3- to 35-mer, 3- to 28-mer, 4- to 25-mer, 5- to 20-mer, 6- to 22-mer, 8- to 20-mer, or 10- to 15-mer.

11. The alginate oligomer for use of any one of claims 1 to 10, wherein the alginate oligomer has at least 85%, at least 90%, at least 95%, or 100% G residues.

12. The alginate oligomer for use of any one of claims 1 to 11, wherein at least 80% of the G residues are arranged in G-blocks.

13. The alginate oligomer for use of any one of claims 1 to 10, wherein at least 70%, at least 80%, at least 85%, at least 90%, at least 95%, at least 99% or 100% of the G and M residues in the oligomer are arranged in MG blocks.

**Patentansprüche**

1. Alginatoligomer zur Verwendung in der Antikoagulationstherapie eines menschlichen oder nichtmenschlichen Wirbelsubjekt mit Risiko von Blutgerinnseln, das eine abnormal dichte Mikrostruktur aufweist, wobei das Alginatoligomer 2 bis 40 Momomerreste aufweist, von den mindestens 80 % Guluronsäure-(G)-Reste sind, und wobei die Blutgerinnsel, die eine anormal dichte Mikrostruktur aufwiesen, Blutgerinnseln sind, die Folgendes aufweisen:

(i) einen Gerinnseldurchlässigkeits-Ks-Wert, der mindestens etwa $1 \times 10^{-9}$ cm$^2$ niedriger ist als der Ks-Wert von Blutgerinnseln eines normalen Subjekts der gleichen Spezies, oder
(ii) ein $d_f$ größer als etwa 1,76 bei dem Gelpunkt.

2. Alginatoligomer zur Verwendung nach Anspruch 1, wobei die Antikoagulationstherapie Behandeln oder Vorbeugen einer Krankheit oder eines gesundheitlichen Zustands ist, die mit Blutgerinnseln in Zusammenhang stehen, die eine anormal dichte Mikrostruktur aufweisen.

3. Alginatoligomer zur Verwendung nach Anspruch 1 oder Anspruch 2, wobei die Inzidenz von Blutgerinnseln, die eine anormal dichte Mikrostruktur aufweisen, in dem Subjekt verringert ist und/oder das Auftreten und/oder die Bildung von Blutgerinnseln, die eine anormal dichte Mikrostruktur aufweisen, in dem Subjekt gehemmt ist.

4. Alginatoligomer zur Verwendung nach einem der Ansprüche 1 bis 3, wobei die Mikrostrukturdichte von Blutgerinnseln in dem Subjekt verringert ist und/oder eine normale Mikrostrukturdichte bei Blutgerinnseln während der Bildung davon im Subjekt gefördert ist.

5. Alginatoligomer zur Verwendung nach Anspruch 2, wobei die Krankheit oder der gesundheitliche Zustand, die mit Blutkoagulation in Zusammenhang stehen, kardiovaskuläre Krankheit, ischämische Herzkrankheit, Koronararterienkrankheit, Venenthrombose, Arterienthrombose, Atherosklerose, Atherothrombose, Venen-Transplantatversagen, Arterien-Transplantatversagen, Schlaganfall, Herzinfarkt, Lungenembolie und Thrombophilie ist.

6. Alginatoligomer zur Verwendung nach einem der Ansprüche 1 bis 5, wobei die Blutgerinnsel, die eine anormal dichte Mikrostruktur aufweisen, Blutgerinnsel sind, die Folgendes aufweisen:

(i) höhere Fibrinogenspiegel;
(ii) höhere glykierte Fibrinogenspiegel;
(iii) dünnere Fibrinfasern;
(iv) größere Fibrinverzweigung;
(v) kürzere Fibrinfasern;
(vi) verringerter Durchlässigkeit; und/oder
(vii) verringerte Porosität,

im Vergleich zu Blutgerinnseln von einem normalen Subjekt der gleichen Spezies.

7. Alginatoligomer zur Verwendung nach einem der Ansprüche 1 bis 6, wobei das Subjekt ausgewählt ist aus einem Subjekt mit

(i) Krebs,
(ii) einer entzündlichen Erkrankung,
(iii) venöser Thromboembolie (z. B. Lungenembolie, tiefe Venenthrombose, zerebrale Sinusvenenthrombose),
(iv) ischämischem Schlaganfall,
(v) Myokardinfarkt,
(vi) Koronararterienkrankheit,
(vii) Stentthrombose,
(viii) Phänomen des Ausbleibens ausreichender Durchblutung nach Entfernen von vaskulärer Okklusion,
(ix) chronisch-obstruktive Lungenerkrankung,
(x) chronisch thromboembolische pulmonale Hypertonie,
(xi) Nierenkrankheit im Endstadium,
(xii) abdominales Aortenaneurysma,
(xiii) rheumatoide Arthritis,
(xiv) residualer Venenverschluss,
(xv) periphere Gefäßkrankheit,
(xvi) Diabetes mellitus,
(xvii) Sepsis oder
(xviii) Sichelzellkrankheit.

8. Alginatoligomer zur Verwendung nach einem der Ansprüche 1 bis 7, wobei das Subjekt

(i) einem chirurgischen Verfahren unterzogen wird, in Kürze unterzogen wird oder vor Kurzem unterzogen wurde
(ii) einer Implantation eines medizinischen Verweil-, chirurgischen oder prosthetischen Elements oder Gewebetransplantation unterzogen wird oder in Kürze unterzogen wird
(iii) eine Implantation eines medizinischen Verweil-, chirurgischen oder prosthetischen Elements oder Gewebetransplantation erhalten hat,
(iv) Dialyse und/oder Apherese unterzogen wird, in Kürze unterzogen wird oder vor Kurzem unterzogen wurde.

9. Alginatoligomer zur Verwendung nach einem der Ansprüche 1 bis 8, wobei das Alginatoligomer einen Polymerisationsgrad (DP) oder einen Zahlendurchschnittsgrad von Polymerisation (Dpn) aufweist von

(i) 2 bis 35, 2 bis 30, 2 bis 25, 2 bis 22, 2 bis 20, 2 bis 18, 2 bis 16 oder 2 bis 14,
(i) 4 bis 35, 4 bis 30, 4 bis 25, 4 bis 22, 4 bis 20, 4 bis 18, 4 bis 16 oder 4 bis 14,
(iii) 6 bis 35, 6 bis 30, 6 bis 25, 6 bis 22, 6 bis 20, 6 bis 18, 6 bis 16 oder 6 bis 14 oder
(iv) 8 bis 35, 8 bis 30, 8 bis 25, 10 bis 25, 10 bis 22, 10 bis 20, 10 bis 18 oder 10 bis 14.

10. Alginatoligomer zur Verwendung nach den Ansprüchen 1 bis 10, wobei das Alginatoligomer ein 2- bis 35-Mer, 2- bis 30-Mer, 3- bis 35-Mer, 3- bis 28-Mer, 4- bis 25-Mer, 5- bis 20-Mer, 6- bis 22-Mer, 8- bis 20-Mer oder 10- bis 15-Mer ist.

**11.** Alginatoligomer zur Verwendung nach einem der Ansprüche 1 bis 10, wobei das Alginatoligomer mindestens 85 %, mindestens 90 %, mindestens 95 %, der 100 % G-Reste aufweist.

**12.** Alginatoligomer zur Verwendung nach einem der Ansprüche 1 bis 11, wobei mindestens 80 % der G-Reste in G-Blöcken angeordnet sind.

**13.** Alginatoligomer zur Verwendung nach einem der Ansprüche 1 bis 10, wobei mindestens 70 %, mindestens 80 %, mindestens 85 %, mindestens 90 %, mindestens 95 %, mindestens 99 % oder 100 % der G- und M-Reste in dem Oligomer in MG-Blöcken angeordnet sind.

**Revendications**

**1.** Oligomère d'alginate pour une utilisation dans le traitement anticoagulant d'un sujet vertébré humain ou non humain présentant un risque de caillots sanguins qui présentent une microstructure anormalement dense, dans lequel ledit oligomère d'alginate présente 2 à 40 résidus de monomère dont au moins 80 % sont des résidus d'acide guluronique (G), et dans lequel lesdits caillots sanguins qui présentent une microstructure anormalement dense sont des caillots sanguins qui présentent

> (i) une valeur Ks de perméabilité de caillot qui est inférieure au moins d'environ $1 \times 10^{-9}$ cm$^2$ à la valeur Ks des caillots sanguins d'un sujet normal de la même espèce, ou
> (ii) un $d_f$ supérieur à environ 1,76 au point de gel.

**2.** Oligomère d'alginate pour une utilisation selon la revendication 1, dans lequel ledit traitement anticoagulant comprend le fait de traiter ou d'empêcher une maladie ou une affection associée à des caillots sanguins qui présentent une microstructure anormalement dense.

**3.** Oligomère d'alginate pour une utilisation selon la revendication 1 ou la revendication 2, dans lequel l'incidence de caillots sanguins qui présentent une microstructure anormalement dense chez le sujet est réduite et/ou l'apparition et/ou la formation de caillots sanguins qui présentent une microstructure anormalement dense chez le sujet est inhibée.

**4.** Oligomère d'alginate pour une utilisation selon l'une quelconque des revendications 1 à 3, dans lequel la densité de microstructure de caillots sanguins chez le sujet est réduite et/ou une densité de microstructure normale dans les caillots sanguins pendant leur formation dans le sujet est favorisée.

**5.** Oligomère d'alginate pour une utilisation selon la revendication 2, dans lequel ladite maladie ou ladite affection associée à la coagulation sanguine est une maladie cardiovasculaire, une cardiopathie ischémique, une maladie coronarienne, une thrombose veineuse, une thrombose artérielle, une athérosclérose, une athérothrombose, une défaillance de greffe veineuse, une défaillance de greffe artérielle, un accident vasculaire cérébral, un infarctus du coeur, une embolie pulmonaire et une thrombophilie.

**6.** Oligomère d'alginate pour une utilisation selon l'une quelconque des revendications 1 à 5, dans lequel lesdits caillots sanguins qui présentent une microstructure anormalement dense sont des caillots sanguins qui présentent

> (i) des niveaux supérieurs de fibrinogène ;
> (ii) des niveaux supérieurs de fibrinogène glyqué ;
> (iii) des fibres de fibrine plus fines ;
> (iv) une plus grande ramification de fibrine ;
> (v) des fibres de fibrine plus courtes ;
> (vi) une perméabilité réduite ; et/ou
> (vii) une porosité réduite,

par rapport aux caillots sanguins d'un sujet normal de la même espèce.

**7.** Oligomère d'alginate pour une utilisation selon l'une quelconque des revendications 1 à 6, dans lequel le sujet est sélectionné parmi un sujet avec

(i) un cancer,

(ii) une maladie inflammatoire,

(iii) une thromboembolie veineuse (par exemple, une embolie pulmonaire, une thrombose veineuse profonde, une thrombose des sinus veineux cérébraux),

(iv) un accident vasculaire cérébral ischémique,

(v) un infarctus du myocarde,

(vi) une maladie coronarienne,

(vii) une thrombose intra-stent,

(viii) un phénomène de non-reflux après retrait d'une occlusion vasculaire,

(ix) une maladie pulmonaire obstructive chronique,

(x) une hypertension pulmonaire thromboembolique chronique,

(xi) une insuffisance rénale terminale,

(xii) un anévrisme de l'aorte abdominale,

(xiii) une arthrite rhumatoïde,

(xiv) une obstruction veineuse résiduelle,

(xv) une maladie artérielle périphérique,

(xvi) le diabète sucré,

(xvii) un sepsis, ou

(xviii) la drépanocytose.

8. Oligomère d'alginate pour une utilisation selon l'une quelconque des revendications 1 à 7, dans lequel le sujet

(i) est en train de subir, sur le point de subir ou a récemment subi une intervention chirurgicale

(ii) est en train de subir ou sur le point de subir l'implantation d'un dispositif médical, chirurgical ou prothétique ou d'une greffe de tissu à demeure

(iii) a reçu un dispositif médical, chirurgical ou prothétique ou une greffe de tissu à demeure,

(iv) est en train de subir, sur le point de subir ou a récemment subi une dialyse et/ou une aphérèse.

9. Oligomère d'alginate pour une utilisation selon l'une quelconque des revendications 1 à 8, dans lequel l'oligomère d'alginate présente un degré de polymérisation (DP), ou un degré de polymérisation moyen en nombre (DPn) de

(i) 2 à 35, 2 à 30, 2 à 25, 2 à 22, 2 à 20, 2 à 18, 2 à 16 ou 2 à 14,

(i) 4 à 35, 4 à 30, 4 à 25, 4 à 22, 4 à 20, 4 à 18, 4 à 16 ou 4 à 14,

(iii) 6 à 35, 6 à 30, 6 à 25, 6 à 22, 6 à 20, 6 à 18, 6 à 16 ou 6 à 14, ou

(iv) 8 à 35, 8 à 30, 8 à 25, 10 à 25, 10 à 22, 10 à 20, 10 à 18, ou 10 à 14.

10. Oligomère d'alginate pour une utilisation selon les revendications 1 à 10, dans lequel l'oligomère d'alginate est un 2- à 35-mer, 2- à 30-mer, 3- à 35-mer, 3- à 28-mer, 4-à 25-mer, 5- à 20-mer, 6- à 22-mer, 8- à 20-mer, ou 10- à 15-mer.

11. Oligomère d'alginate pour une utilisation selon l'une quelconque des revendications 1 à 10, dans lequel l'oligomère d'alginate présente au moins 85 %, au moins 90 %, au moins 95 % ou 100 % de résidus de G.

12. Oligomère d'alginate pour une utilisation selon l'une quelconque des revendications 1 à 11, dans lequel au moins 80 % des résidus de G sont agencés en blocs G.

13. Oligomère d'alginate pour une utilisation selon l'une quelconque des revendications 1 à 10, dans lequel au moins 70 %, au moins 80 %, au moins 85 %, au moins 90 %, au moins 95 %, au moins 99 % ou 100 % des résidus de G et M dans l'oligomère sont agencés en blocs MG.

# Figure 1

A

B

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006111758 A
- WO 9409124 A
- WO 2004011628 A
- WO 2007039754 A
- WO 2008125828 A
- WO 2009068841 A
- WO 201013957 A
- WO 2015092437 A
- WO 2007039760 A
- US 6121441 A
- WO 2005023176 A

### Non-patent literature cited in the description

- **EVANS PA et al.** *Blood*, 2010, vol. 116 (17), 3341-3346
- **LAWRENCE, MJ et al.** *Br J Haematol.*, 2014, vol. 168, 571-575
- **LAWRENCE, MJ et al.** *Thromb Res.*, 2014, vol. 134 (2), 488-94
- **LAWRENCE MJ et al.** *Atherosclerosis*, 2015, vol. 240, 402-407
- **STANFORD, SN et al.** *BMC Neurology*, 2015, vol. 15, 35
- **DAVIES, NA et al.** *Thromb Haemost.*, 2015, vol. 114 (6), 1251-9
- **DAVIES, GR et al.** *Intensive Care Medicine*, 2016, vol. 42 (12), 1990-1998
- **BARADET, T. C**. *Biophysical journal*, 1995, vol. 68, 1551-1560
- **FAN, K. et al.** *Journal of Visualised Experiments*, 2015, vol. 98, e52019
- **WUFSUS, AR et al.** *Biophysical Journal*, 2013, vol. 104, 1812-1823
- **SPERO, RC et al.** *Biophysical Journal*, 2011, vol. 101, 943-950
- **PIETERS M et al.** *Journal of Thrombosis and Haemostasis*, 2012, vol. 10, 2179-2181
- **JÖRNESKOG G et al.** *Diabetologia*, 1996, vol. 39 (12), 1519-1523
- **CARR, M.** *Journal of Diabetes and Its Complications*, 2001, vol. 15, 44-54
- **MILLS JD et al.** *Circulation*, 2002, vol. 106 (15), 1938-1942
- **GIMMESTAD, M et al.** *Journal of Bacteriology*, 2003, vol. 185 (12), 3515-3523
- **EVANS ; LAWRENCE**. *Br J Haematol*, 2014
- **LAWRENCE**. *Thromb Res*, 2014
- **LAWRENCE**. *Stanford*, 2015
- Handbooks of Hydrocolloids. CRC, 2000
- **ERTESVÅG, H. et al.** *Metabolic Engineering*, 1999, vol. 1, 262-269
- **REMMINGHORST ; REHM**. *Biotechnology Letters*, 2006, vol. 28, 1701-1712
- **GIMMESTAD, M. et al.** *Journal of Bacteriology*, 2006, vol. 188 (15), 5551-5560
- **STRUGALA et al.** Gums and Stabilisers for the Food Industry. The Royal Society of Chemistry, 2004, vol. 12, 84-94
- Remington's Pharmaceutical Sciences. Mack Publishing Co, 1975, 1405-1412, 1461-1487
- The National Formulary XIV. American Pharmaceutical Association, 1975